# EUROPÄISCHE PATENTSCHRIFT

(11) **EP 1 741 149 B1**
(45) Veröffentlichungstag und Bekanntmachung des Hinweises auf die Patenterteilung: **24.12.2008**
(21) Anmeldenummer: 05744529.8
(22) Anmeldetag: 26.04.2005
(51) Int. Cl.: H01L 51/30, C09K 11/06, H05B 33/14, C08G 61/02, C08G 61/12, C08L 65/00, C07C 25/22, C07F 5/02, C07C 211/60, C07C 25/24

(54) **ELEKTROLUMINESZIERENDE POLYMERE UND DEREN VERWENDUNG**
ELECTROLUMINESCENT POLYMERS AND USE THEROF
POLYMERES ELECTROLUMINESCENTS ET LEUR UTILISATION

(30) Priorität: 26.04.2004 DE 102004020298
(43) Veröffentlichungstag der Anmeldung: 10.01.2007
(73) Patentinhaber: Merck Patent GmbH, 64293 Darmstadt (DE)
(72) Erfinder: BÜSING, Arne, 65929 Frankfurt (DE); HEUN, Susanne, 65812 Bad Soden (DE); TÜRK, Silke, 56410 Montabaur (DE); LESKE, Corinna, 60529 Frankfurt (DE)
(86) Internationale Anmeldenummer: PCT/EP2005/004448
(87) Internationale Veröffentlichungsnummer: WO 2005/104264

(56) Entgegenhaltungen:
- EP-A- 1 329 474
- WO-A-20/04037887
- WELDON G. BROWN, BEN A. BLUESTEIN: "Conversion of 2,7-Dibromofluorene to 2,7-Dibromophenanthrene" JOURNAL OF THE AMERICAN CHEMICAL SOCIETY, Bd. 65, 1943, Seiten 1235-1236, XP002339237
- CHARLES K. BRADSHER, LEO E. BEAVERS, N. TOKURA: "Aromatic Cyclodehydration. XXXIII. 2,7-Disubstituted Phenanthrenes" JOURNAL OF THE AMERICAN CHEMICAL SOCIETY, Bd. 78, 1956, Seiten 3196-3198, XP002339238
- LEE C. WASHBURN, D.E. PEARSON: "Potential Antimalarials. 8. Some 10-Substituted 9-Phenanthrenemethanols" JOURNAL OF MEDICINAL CHEMISTRY, Bd. 17, Nr. 7, 1974, Seiten 676-682, XP002339239
- V. N. BOCHENKOV: "Synthesis of some 2,7- and 2,5-Disubstituted Phenanthrenes" JOURNAL OF ORGANIC CHEMISTRY OF THE USSR, Bd. 12, 1976, Seiten 2355-2357, XP009051835

## Beschreibung

Seit ca. 13 Jahren läuft eine breit angelegte Forschung zur Kommerzialisierung von Anzeige- und Beleuchtungselementen auf Basis polymerer (organischer) Leuchtdioden (PLEDs). Ausgelöst wurde diese Entwicklung durch die Grundlagenentwicklungen, welche in WO 90/13148 offenbart sind. Seit kurzem ist ein erstes, wenn auch einfaches, Produkt (eine kleine Anzeige in einem Rasierapparat der Fa. PHILIPS N.V.) auf dem Markt erhältlich. Allerdings sind immer noch deutliche Verbesserungen der verwendeten Materialien nötig, um diese Displays zu einer echten Konkurrenz zu den derzeit marktbeherrschenden Flüssigkristallanzeigen (LCD) zu machen.

Es ist für die Erzeugung aller drei Emissionsfarben nötig, bestimmte Comonomere in die entsprechenden Polymere einzupolymerisieren (vgl. z. B. WO 00/46321, WO 03/020790 und WO 02/077060). So ist dann in der Regel, ausgehend von einem blau emittierenden Grundpolymer ("backbone"), die Erzeugung der beiden anderen Primärfarben Rot und Grün möglich.

Als Polymere für vollfarbige Anzeigeelemente (Full-Colour-Displays) wurden bereits verschiedene Materialklassen vorgeschlagen bzw. entwickelt. So kommen Poly-FluorenDerivate in Betracht; des Weiteren sind auch Poly-Spirobifluoren-, Poly-Dihydrophenanthren- und Poly-Indenofluoren-Derivate eine Möglichkeit. Auch Polymere, die eine Kombination der beiden erstgenannten Strukturelemente enthalten, wurden bereits vorgeschlagen. Im Allgemeinen sind für derartigen Einsatz Polymere, welche Poly-paraphenylen (PPP) als Strukturelement enthalten, möglich.

Die Polymere gemäß dem Stand der Technik zeigen zum Teil schon gute Eigenschaften in der Anwendung in PLEDs. Trotz der bereits erzielten Fortschritte entsprechen diese Polymere allerdings noch nicht den Anforderungen, die an sie für hochwertige Anwendungen gestellt werden. Insbesondere ist die Lebensdauer der grün und vor allem der blau emittierenden Polymere für viele Anwendungen noch nicht ausreichend, ebenso wie die Effizienz der rot emittierenden Polymeren. Weiterhin ist bei vielen Polymeren blau emittierenden gemäß dem Stand der Technik die Emissionsfarbe noch nicht ausreichend tiefblau.

Es wurde nun überraschend gefunden, dass eine neue Klasse von Polymeren sehr gute und den o. g. Stand der Technik übertreffende Eigenschaften aufweist. Diese Polymere und deren Verwendung in PLEDs sind daher Gegenstand der vorliegenden Erfindung. Die neuen Struktureinheiten eignen sich insbesondere als Polymer-Grundgerüst, aber je nach Substitutionsmuster auch als Lochleiter, Elektronenleiter oder Emitter.

Die Verwendung von Phenanthrenen in elektrolumineszierenden Polymeren wurde schon gelegentlich allgemein erwähnt, z. B. in WO 02/077060, WO 03/020790 und WO 05/014689. Allerdings ist dort nur allgemein aufgezählt, dass diese Strukturelemente, ebenso wie eine große Vielzahl weiterer Monomere, als mögliche weitere.Elemente, neben dem eigentlichen Polymer-Grundgerüst, vorhanden sein können. Besondere Vorteile dieser Einheiten sind nicht beschrieben. Außerdem ist nur ganz allgemein beschrieben, dass diese mit nicht-aromatischen Substituenten substituiert oder unsubstituiert sein können. Die Verwendung unsubstituierter Phenanthren-Einheiten führt jedoch zu unlöslichen Polymeren, so dass diese Einheiten höchstens in sehr geringem Anteil verwendet werden können. Welche Substituenten allerdings besonders geeignet sind und an welchen Positionen der Phenanthren-Einheit diese Substituenten bevorzugt gebunden sein sollten, geht aus diesen Beschreibungen nicht hervor. Ebenso wenig geht daraus hervor; dass sich die neuen Struktureinheiten besonders eignen, zu höherem Anteil im Polymer eingesetzt zu werden, da sie im Stand der Technik nur als Comonomere in vergleichsweise geringen Anteilen erwähnt sind. Es ist also auch für den Fachmann nicht ersichtlich, wie diese Einheiten nutzbringend in elektrolumineszierenden Polymeren verwendet werden könnten. Daher ist die allgemeine Aufführung von unsubstituierten oder beliebig substituierten Phenanthren-Einheiten als zufällige Offenbarung zu bewerten.

Die Substitution der Phenanthren-Einheiten in 9- bzw. 9,10-Position und die Verknüpfung im Polymer in der 2,7-Position hat sich überraschend als besonders geeignet im Vergleich zur Substitution in anderen Positionen der Phenanthren-Einheit erwiesen. Diese Bevorzugung lässt sich durch die besonders gute synthetische Zugänglichkeit der in diesen Positionen substituierten Einheiten begründen, aber auch durch die besseren optischen und elektronischen Eigenschaften.

Gegenstand der Erfindung sind Polymere, enthaltend mindestens 5 mol%, bevorzugt mindestens 10 mol%, besonders bevorzugt mindestens 30 mol%, ganz besonders bevorzugt mindestens 50 mol% Einheiten gemäß Formel (1), 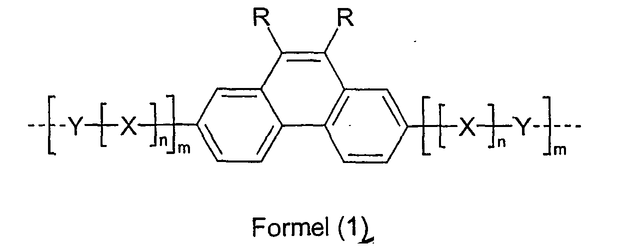 wobei die verwendeten Symbole und Indizes folgende Bedeutung besitzen:
- R: ist bei jedem Auftreten gleich eine geradkettige, verzweigte oder cyclische Alkylkette mit 1 bis 40 C-Atomen, die durch R¹ substituiert sein kann, und in der auch ein oder mehrere nicht benachbarte C-Atome durch N-R¹, O, S, O-CO-O, CO-O, -CR¹=CR¹- oder -C≡C- ersetzt sein können, mit der Maßgabe, dass die Heteroatome nicht direkt an die Phenanthren-Einheit gebunden sind, und in der auch ein oder mehrere H-Atome durch F, Cl, Br, I oder CN ersetzt sein können, oder ein aromatisches oder heteroaromatisches Ringsystem mit 2 bis 40 C-Atomen, welches auch durch einen oder mehrere Reste R¹ substituiert sein kann, oder eine Kombination aus mehreren dieser Systeme; dabei können die beiden Reste R miteinander auch ein weiteres mono- oder polycyclisches, aliphatisches Ringsystem bilden;
oder verschieden H, eine geradkettige, verzweigte oder cyclische Alkylkette mit 1 bis 40 C-Atomen, die durch R¹ substituiert sein kann, und in der auch ein oder mehrere nicht benachbarte C-Atome durch N-R¹, O, S, O-CO-O, CO-O, -CR¹=CR¹- oder -C≡C- ersetzt sein können, mit der Maßgabe, dass die Heteroatome nicht direkt an die Phenanthren-Einheit gebunden sind, und in der auch ein oder mehrere H-Atome durch F, Cl, Br, I oder CN ersetzt sein können, oder ein aromatisches oder heteroaromatisches Ringsystem mit 2 bis 40 C-Atomen, welches auch durch einen oder mehrere Reste R¹ substituiert sein kann, oder eine Kombination aus mehreren dieser Systeme; dabei können die beiden Reste R miteinander auch ein weiteres mono- oder polycyclisches, aliphatisches Ringsystem bilden;
- X: ist bei jedem Auftreten gleich oder verschieden -CR¹=CR¹-, -C≡C- oder N-Ar;
- Y: ist bei jedem Auftreten gleich oder verschieden ein bivalentes aromatisches oder heteroaromatisches Ringsystem mit 2 bis 40 C-Atomen, welches durch einen oder mehrere Reste R¹ substituiert oder unsubstituiert sein kann;
- R¹: ist bei jedem Auftreten gleich oder verschieden H, eine geradkettige, verzweigte oder cyclische Alkyl- oder Alkoxykette mit 1 bis 22 C-Atomen, in der auch ein oder mehrere nicht benachbarte C-Atome durch N-R², O, S, O-CO-O, CO-O, -CR²=CR²-, -C≡C- ersetzt sein können und in der auch ein oder mehrere H-Atome durch F, Cl, Br, I oder CN ersetzt sein können, oder eine Aryl-, Heteroaryl-, Aryloxy- oder Heteroaryloxygruppe mit 5 bis 40 C-Atomen, welche auch durch einen oder mehrere nicht-aromatische Reste R¹ substituiert sein kann; dabei können auch zwei oder mehrere der Reste R¹ miteinander und/oder mit R ein Ringsystem bilden; oder F, Cl, Br, I, CN, N(R²)₂, Si(R²)₃ oderB(R²)₂;
- R²: ist bei jedem Auftreten gleich oder verschieden H oder ein aliphatischer oder aromatischer Kohlenwasserstoffrest mit 1 bis 20 C Atomen;
- Ar: ist bei jedem Auftreten gleich oder verschieden ein monovalentes aromatisches oder heteroaromatisches Ringsystem mit 2 bis 40 C-Atomen, welches mit R¹ substituiert oder unsubstituiert sein kann;
- n: ist bei jedem Auftreten gleich oder verschieden 0 oder 1;
- m: ist bei jedem Auftreten gleich oder verschieden 0,1 oder 2;
die gestrichelte Bindung bedeutet dabei die Verknüpfung im Polymer; sie soll hier keine Methylgruppe darstellen.

Auch wenn dies aus der Beschreibung hervorgeht, sei hier nochmals explizit darauf verwiesen, dass die Struktureinheiten gemäß Formel (1) unsymmetrisch substituiert sein können, d. h. dass an einer Einheit unterschiedliche Substituenten R bzw. R¹ vorhanden sein können, bzw. dass die Substituenten X und Y, soweit vorhanden, unterschiedlich sind oder auch nur einseitig auftreten.

Unter einem aromatischen bzw. heteroaromatischen Ringsystem im Sinne dieser Erfindung soll ein System verstanden werden, das nicht notwendigerweise nur aromatische bzw. heteroaromatische Gruppen enthält, sondern in dem auch mehrere aromatische bzw. heteroaromatische Gruppen durch eine kurze nicht-aromatische Einheit (< 10 % der von H verschiedenen Atome, bevorzugt < 5 % der von H verschiedenen Atome), wie beispielsweise sp³-hybridisierter C, O, N, etc., unterbrochen sein können. So sollen also beispielsweise auch Systeme wie 9,9'-Spirobifluoren, 9,9-Diarylfluoren, Triarylamin, etc. als aromatische Ringsysteme verstanden werden. Dabei enthält ein aromatisches Ringsystem mindestens 6 C-Atome und ein heteroaromatisches Ringsystem mindestens 2 C-Atome und mindestens ein Heteroatom, bevorzugt ausgewählt aus N, O und/oder S, mit der Maßgabe, dass die Summe aus C-Atomen und Heteroatomen mindestens 5 ergibt.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einer C₂-C₂₄ Aryl- oder Heteroarylgruppe, die je nach Verwendung monovalent oder bivalent sein kann, die noch jeweils mit den oben genannten Resten R¹ substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Chrysen, Perylen, Fluoranthen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1.,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter aromatischen Ringsystemen werden weiterhin insbesondere Biphenylen, Terphenylen, Fluoren, Spirobifluoren, Dihydrophenanthren, Dihydropyren, Tetrahydropyren oder cis- oder trans-Indenofluoren verstanden.

In einem Aspekt der Erfindung handelt es sich um konjugierte Polymere. In einem weiteren Aspekt der Erfindung handelt es sich um nicht-konjugierte Polymere. In nochmals einem weiteren Aspekt der Erfindung handelt es sich um teilkonjugierte Polymere. Bevorzugt sind konjugierte oder teilkonjugierte Polymere.

Konjugierte Polymere im Sinne dieser Erfindung sind Polymere, die in der Hauptkette hauptsächlich sp²-hybridisierte Kohlenstoffatome, die auch durch entsprechende Heteroatome ersetzt sein können, enthalten. Dies bedeutet im einfachsten Fall abwechselndes Vorliegen von Doppel- und Einfachbindungen in der Hauptkette. Hauptsächlich meint, dass natürlich auftretende Defekte, die zu Konjugationsunterbrechungen führen, den Begriff "konjugiertes Polymer" nicht entwerten. Des Weiteren wird in diesem Anmeldetext ebenfalls als konjugiert bezeichnet, wenn sich in der Hauptkette beispielsweise Arylamineinheiten und/oder bestimmte Heterocyclen (d. h. Konjugation über N-, O- oder S-Atome) und/oder metallorganische Komplexe (d. h. Konjugation über das Metallatom) befinden. Hingegen würden Einheiten wie beispielsweise einfache Alkylbrücken, (Thio)Ether-, Ester-, Amid- oder Imidverknüpfungen eindeutig als nicht-konjugierte Segmente definiert. Unter einem teilkonjugierten Polymer soll ein Polymer verstanden werden, in dem längere konjugierte Abschnitte in der Hauptkette durch nicht-konjugierte Abschnitte unterbrochen sind, bzw. das längere konjugierte Abschnitte in den Seitenketten eines in der Hauptkette nicht-konjugierten Polymers enthält.

Die erfindungsgemäßen Polymere können neben Einheiten gemäß Formel (1) noch weitere Strukturelemente enthalten. Dies sind u. a. solche, wie sie in WO 02/077060 und WO 05/014689 offenbart und umfangreich aufgelistet sind. Die weiteren Struktureinheiten können beispielsweise aus den im Folgenden beschriebenen Klassen stammen:
- Gruppe 1:: Einheiten, welche die Lochinjektions- und/oder -transporteigenschaften der Polymere erhöhen;
- Gruppe 2:: Einheiten, welche die Elektroneninjektions- und/oder -transporteigenschaften der Polymere erhöhen;
- Gruppe 3:: Einheiten, die Kombinationen von Einzeleinheiten der Gruppe 1 und Gruppe 2 aufweisen;
- Gruppe 4:: Einheiten, welche die Emissionscharakteristik insoweit verändern, dass Elektrophosphoreszenz statt Elektrofluoreszenz erhalten werden kann;
- Gruppe 5:: Einheiten, welche den Übergang vom Singulett- zum Triplettzustand verbessern;
- Gruppe 6:: Einheiten, welche die Morphologie öder auch die Emissionsfarbe der resultierenden Polymere beeinflussen;
- Gruppe 7:: Einheiten, welche typischerweise als Backbone verwendet werden.

Bevorzugte erfindungsgemäße Polymere sind solche, bei denen mindestens ein Strukturelement Ladungstransporteigenschaften aufweist, d. h. die Einheiten aus den Gruppen 1 und/oder 2 enthalten.

Strukturelemente aus der Gruppe 1, die Lochtransporteigenschaften aufweisen, sind beispielsweise Triarylamin-, Benzidin-, Tetraaryl-para-phenylendiamin-, Triarylphosphin-, Phenothiazin-, Phenoxazin-, Dihydrophenazin-, Thianthren-, Dibenzo-p-dioxin-, Phenoxathiin-, Carbazol-, Azulen-, Thiophen-, Pyrrol- und Furanderivate und weitere O-, S- oder N-haltige Heterocyclen mit hoch liegendem HOMO (HOMO = höchstes besetztes Molekülorbital); bevorzugt führen diese Arylamine und Heterocyclen zu einem HOMO im Polymer von mehr als -5.8 eV (gegen Vakuumlevel), besonders bevorzugt von mehr als -5.5 eV.

Strukturelemente aus Gruppe 2, die Elektronentransporteigenschaften aufweisen, sind beispielsweise Pyridin-, Pyrimidin-, Pyridazin-, Pyrazin-, Oxadiazol-, Chinolin-, Chinoxalin-, Benzothiadiazol- und Phenazinderivate, aber auch Triarylborane und weitere O-, S- oder N-haltige Heterocyclen mit niedrig liegendem LUMO (LUMO = niedrigstes unbesetztes Molekülorbital); bevorzugt führen diese Einheiten im Polymer zu einem LUMO von weniger als -2.7 eV (gegen Vakuumlevel), besonders bevorzugt von weniger als -3.0 eV.

Es kann bevorzugt sein, wenn in den erfindungsgemäßen Polymeren. Einheiten aus Gruppe 3 enthalten sind, in denen Strukturen, welche die Lochmobilität und welche die Elektronenmobilität erhöhen (also Einheiten aus Gruppe 1 und 2), direkt aneinander gebunden sind. Einige dieser Einheiten können als Emitter dienen und verschieben die Emissionsfarbe ins Grüne, Gelbe oder Rote; ihre Verwendung eignet sich also beispielsweise für die Erzeugung anderer Emissionsfarben aus ursprünglich blau emittierenden Polymeren.

Struktureinheiten gemäß Gruppe 4 sind solche, welche auch bei Raumtemperatur mit hoher Effizienz aus dem Triplettzustand Licht emittieren können, also Elektrophosphoreszenz statt Elektrofluoreszenz zeigen, was häufig eine Steigerung der Energieeffizienz bewirkt. Hierfür eignen sich zunächst Verbindungen, welche Schweratome mit einer Ordnungszahl von mehr als 36 enthalten. Besonders geeignet sind Verbindungen, welche d- oder f-Übergangsmetalle beinhalten, die die o. g. Bedingung erfüllen. Ganz besonders bevorzugt sind hier entsprechende Struktureinheiten, welche Elemente der Gruppe 8 bis 10 (Ru, Os, Rh, Ir, Pd, Pt) enthalten. Als Struktureinheiten für die erfindungsgemäßen Polymeren kommen hier z. B. verschiedene Komplexe in Frage, welche beispielsweise in den Anmeldeschriften WO 02/068435, WO 02/081488, EP 1239526 und WO 04/026886 beschrieben sind. Entsprechende Monomere sind in WO 02/068435 und in der nicht offen gelegten Anmeldung DE 10350606.3 beschrieben.

Strukturelemente der Gruppe 5 sind solche, welche den Übergang vom Singulett- zum Triplettzustand verbessern und welche, unterstützend zu den Strukturelementen der Gruppe 4 eingesetzt, die Phosphoreszenzeigenschaften dieser Strukturelemente verbessern. Hierfür kommen insbesondere Carbazol- und überbrückte Carbazoldimereinheiten in Frage, wie in WO 04/070772 und WO 04/113468 beschrieben. Weiterhin kommen hierfür Ketone, Phosphinoxide; Sulfoxide und ähnliche Verbindungen in Frage, wie in der nicht offen gelegten Anmeldung DE 10349033.7 beschrieben.

Strukturelemente der Gruppe 6, die die Morphologie oder auch die Emissionsfarbe der Polymere beeinflussen, sind neben den oben genannten solche, die mindestens noch eine weitere aromatische oder eine andere konjugierte Struktur aufweisen, welche nicht unter die o. g. Gruppen fällt, d. h. die die Ladungsträgermobilität nur wenig beeinflusst, die keine metallorganischen Komplexe sind oder die keinen Einfluss auf den Singulett-Triplett-Übergang haben. Derartige Strukturelemente können die Morphologie, aber auch die Emissionsfarbe der resultierenden Polymere beeinflussen. Je nach Einheit können sie daher auch als Emitter eingesetzt werden. Bevorzugt sind dabei aromatische Strukturen mit 6 bis 40 C-Atomen oder auch Tolan-, Stilben- oder Bisstyrylarylenderivate, die jeweils mit einem oder mehreren Resten R¹ substituiert sein können. Besonders bevorzugt ist dabei der Einbau von 1,4-Phenylen-, 1,4-Naphthylen-, 1,4- oder 9,10-Anthrylen-, 1,6- oder 2,7- oder 4,9-Pyrenylen-, 3,9- oder 3,10- Perylenylen-, 4,4'-Biphenylylen-, 4,4"-Terphenylylen-, 4,4'-Bi-1,1'-naphthylylen-, 4,4'-Tolanylen-, 4,4'-Stilbenylen- oder 4,4"-Bisstyrylarylenderivaten.

Strukturelemente der Gruppe 7 sind Einheiten, die aromatische Strukturen mit 6 bis 40 C-Atomen beinhalten, welche typischerweise als Polymergrundgerüst (Backbone) verwendet werden. Dies sind beispielsweise 4,5-Dihydropyrenderivate, 4,5,9,10-Tetrahydro-pyrenderivate, Fluorenderivate, 9,9'-Spirobifluorenderivate, 9,10-Dihydrophenanthrenderivate, 5,7-Dihydrodibenzo-oxepinderivate und cis- und translndenofluorenderivate. Da jedoch der Anteil an Einheiten gemäß Formel (1) ganz besonders bevorzugt mindestens 50 mol% beträgt, werden diese Strukturelemente hier nicht bevorzugt als das hauptsächliche Polymergrundgerüst verwendet.

Bevorzugt sind erfindungsgemäße Polymere, die gleichzeitig neben Struktureinheiten gemäß Formel (1) zusätzlich noch eine oder mehrere Einheiten ausgewählt aus den Gruppen 1 bis 7 enthalten. Es kann ebenfalls bevorzugt sein, wenn gleichzeitig mehr als eine Struktureinheit aus einer Gruppe vorliegt. Bevorzugt beträgt der Anteil der Einheiten gemäß Formel (1) mindestens 10 mol%, besonders bevorzugt mindestens 30 mol%, ganz besonders bevorzugt mindestens 50 mol%. Diese Bevorzugung gilt vor allem, wenn es sich bei den Einheiten gemäß Formel (1) um das Polymer-Grundgerüst handelt. Bei anderen Funktionen können andere Anteile bevorzugt sein, beispielsweise ein Anteil in der Größenordnung von 5 bis 20 mol%, wenn es sich um den Lochleiter bzw. den Emitter in einem elektrolumineszierenden Polymer handelt. Für andere Anwendungen, beispielsweise für organische Transistoren, kann der bevorzugte Anteil nochmals unterschiedlich sein, beispielsweise bis zu 100 mol%, wenn es sich um loch- oder elektronenleitende Einheiten handelt.

Bevorzugt sind erfindungsgemäße Polymere, die außer Struktureinheiten gemäß Formel (1) noch mindestens eine Struktureinheit aus den oben genannten Gruppen enthalten. Besonders bevorzugt sind mindestens zwei Struktureinheiten aus unterschiedlichen der oben genannten Klassen. Ganz besonders bevorzugt ist eine dieser Struktureinheiten aus der Gruppe der lochleitenden Einheiten ausgewählt, und die andere Gruppe ist eine emittierende Einheit, wobei diese beiden Funktionen (Lochleitung und Emission) auch von derselben Einheit übernommen werden können.

Einheiten gemäß Formel (1) sind auch insbesondere geeignet zur Synthese weiß emittierenden Copolymere. Diese enthalten bevorzugt einen ausreichend kleinen Anteil grün und rot emittierender Einheiten, so dass insgesamt weiße Emission resultiert. Wie weiß emittierende Copolymere synthetisiert werden können, ist im Detail in der nicht offen gelegten Anmeldung DE 10343606.5 beschrieben.

Die erfindungsgemäßen Polymere weisen bevorzugt 10 bis 10000, besonders bevorzugt 50 bis 5000, ganz besonders bevorzugt 50 bis 2000 Wiederholeinheiten auf.

Die nötige Löslichkeit der Polymere wird v. a. durch die Substituenten R bzw. R¹ an den Einheiten gemäß Formel (1) sowie gegebenenfalls an weiteren anwesenden Einheiten gewährleistet. Falls weitere Substituenten vorhanden sind, tragen auch diese zur Löslichkeit bei.
Um ausreichende Löslichkeit zu gewährleisten, ist es bevorzugt, dass im Durchschnitt pro Wiederholeinheit mindestens 2 nicht-aromatische C-Atome in den Substituenten vorhanden sind. Bevorzugt sind dabei mindestens 4, besonders bevorzugt mindestens 6 C-Atome. Einzelne dieser C-Atome können auch durch O oder S ersetzt sein. Dies kann aber durchaus bedeuten, dass ein gewisser Anteil von Wiederholeinheiten keine weiteren nicht-aromatischen Substituenten trägt.
Um die Morphologie des Films nicht zu verschlechtern, ist es bevorzugt, keine langkettigen Substituenten mit mehr als 12 C-Atomen in einer linearen Kette zu haben, bevorzugt keine mit mehr als 8 C-Atomen, besonders bevorzugt keine mit mehr als 6 C-Atomen.
Nicht-aromatische C-Atome sind, wie beispielsweise in der Beschreibung für R und R¹ in Formel (1), in entsprechenden geradkettigen, verzweigten oder cyclischen Alkyl- oder Alkoxyketten enthalten.

Bevorzugt sind erfindungsgemäße Polymere, bei denen das Symbol R, gleich oder verschieden bei jedem Auftreten, für eine geradkettige, verzweigte oder cyclische Alkylkette mit 2 bis 15 C-Atomen, in der auch ein oder mehrere nicht benachbarte C-Atome durch N-R¹, O, S, O-CO-O, CO-O, -CH=CH- oder -C≡C- ersetzt sein können, mit der Maßgabe, dass die Heteroatome nicht direkt an die Phenanthren-Einheit gebunden sind, und in der auch ein oder mehrere H-Atome durch F oder CN ersetzt sein können, oder eine aromatische oder heteroaromatische Gruppe mit 4 bis 20 C-Atomen, welche auch durch einen oder mehrere nicht-aromatische Reste R¹ substituiert sein kann, oder eine Kombination mehrerer dieser Systeme steht. Dabei können die beiden Reste R zusammen auch ein weiteres mono- oder polycyclisches, aliphatisches Ringsystem bilden. Besonders bevorzugt steht R, gleich oder verschieden bei jedem Auftreten, für eine geradkettige, verzweigte oder cyclische Alkylkette mit 4 bis 8 C-Atomen, besonders bevorzugt eine verzweigte Alkylkette, in der auch ein oder mehrere nicht benachbarte C-Atome durch N-R¹, O, S, -CR¹=CR¹- oder -C≡C- ersetzt sein können, mit der Maßgabe, dass diese nicht direkt an die Phenanthren-Einheit angrenzen, und in der auch ein oder mehrere H-Atome durch F ersetzt sein können, oder eine Aryl- oder Heteroarylgruppe mit 5 bis 10 C-Atomen, die durch R¹ substituiert oder unsubstituiert sein kann; dabei können die beiden Reste R zusammen auch ein weiteres mono- oder polycyclisches Ringsystem bilden.

Bevorzugt sind weiterhin erfindungsgemäße Polymere, bei denen das Symbol X, gleich oder verschieden bei jedem Auftreten, für -CH=CH-, -C≡C- oder N-Ar steht.

Bevorzugt sind weiterhin erfindungsgemäße Polymere, bei denen das Symbol Y, gleich oder verschieden bei jedem Auftreten, für ein bivalentes aromatisches oder heteroaromatisches Ringsystem mit 4 bis 25 C-Atomen, welches durch einen oder mehrere Reste R¹ substituiert sein kann, steht. Besonders bevorzugt steht Y, gleich oder verschieden bei jedem Auftreten, für ein bivalentes aromatisches oder heteroaromatisches Ringsystem mit 6 bis 16 C-Atomen oder Spirobifluoren, welches jeweils durch einen oder mehrere nicht-aromatische Reste R¹ substituiert sein kann.

Bevorzugt sind weiterhin erfindungsgemäße Polymere, bei denen das Symbol Ar, gleich oder verschieden bei jedem Auftreten, für ein monovalentes aromatisches oder heteroaromatisches Ringsystem mit 4 bis 25 C-Atomen, welches mit R¹ substituiert oder unsubstituiert sein kann, steht. Besonders bevorzugt steht Ar, gleich oder verschieden bei jedem Auftreten, für eine monovalente Aryl- oder Heteroarylgruppe mit 4 bis 16 C-Atomen, welche mit nicht-aromatischen Resten R¹ substituiert sein kann.

Bevorzugt sind weiterhin erfindungsgemäße Polymere, bei denen der Index m, gleich oder verschieden bei jedem Auftreten, 0 oder 1 ist.

Je nach Substitutionsmuster eignen sich die Einheiten gemäß Formel (1) für verschiedene Funktionen im Polymer. So können sie bevorzugt als (elektronenleitendes) Polymer-Grundgerüst, als Lochleiter oder als Emitter eingesetzt werden. Welche Verbindungen sich insbesondere für welche Funktion eignen, ist vor allem durch die Substituenten X und Y beschrieben. Auch die Substituenten R haben einen Einfluss auf die elektronischen - Eigenschaften der Einheiten gemäß Formel (1).

So gilt für die Verwendung als Polymer-Grundgerüst bevorzugt:
- n: ist bei jedem Auftreten gleich 0,
d. h. es handelt sich um eine rein aromatische Struktureinheit.

Für die Verwendung von Einheiten gemäß Formel (1) als lochtransportierende Einheiten gilt bevorzugt:
- n: ist bei jedem Auftreten gleich oder verschieden 0 oder 1, wobei mindestens ein n = 1 ist;
- m: ist bei jedem Aufteten gleich oder verschieden 0, 1 oder 2, wobei m ungleich 0 ist, wenn das entsprechende n = 1 ist;
- X: ist bei jedem Auftreten N-Ar;
d. h. es handelt sich um Triarylaminderivate des Phenanthrens.
Weiterhin können Einheiten gemäß Formel (1) auch als lochtransportierende Einheiten verwendet werden, wenn der Index n = 0 ist, falls mindestens einer der Reste R (bzw. an R gebundene Reste R¹) mindestens eine Diarylamin-Gruppe enthalten.

Für die Verwendung von Einheiten gemäß Formel (1) als Emitter gilt bevorzugt:
- n: ist bei jedem Auftreten gleich oder verschieden 0 oder 1, wobei mindestens ein n = 1 ist;
- m: ist bei jedem Auftreten gleich oder verschieden 0, 1 oder 2, wobei m ungleich 0 ist, wenn das entsprechende n = 1 ist;
- X: ist bei jedem Auftreten gleich oder verschieden -CR¹=CR¹-, -C=C- oder N-Ar, wobei mindestens ein X gleich -CR¹=CR¹- oder -C≡C- ist,
d. h. es handelt sich um Diarylvinylen- oder Diarylacetylenderivate im weitesten Sinne, die auch noch zusätzlich Triarylamineinheiten enthalten können.
Weiterhin können Einheiten gemäß Formel (1) auch als emittierend Einheiten verwendet werden, wenn der Index n = 0 ist, falls mindestens einer der Reste R (bzw. an R gebundene Reste R¹) mindestens eine Diarylvinylen- oder Diarylacetylen-Gruppe enthalten.

Weiterhin bevorzugt sind Einheiten gemäß Formel (1), die in den 9,10-Positionen der Phenanthren-Einheiten symmetrisch substituiert sind. Diese Bevorzugung ist durch die bessere synthetische Zugänglichkeit der Monomere zu begründen. Bevorzugt gilt also, dass in einer Einheit gemäß Formel (1) alle R gleich und besonders bevorzugt auch gleich substituiert sind. Diese Bevorzugung schließt nicht aus, dass die Substituenten X und Y nur einseitig auftreten bzw. auch unterschiedlich sein können.

Beispiele für bevorzugte Einheiten gemäß Formel (1) sind Strukturen gemäß den abgebildeten Beispielen 1 bis 36,wobei die Verknüpfung im Polymer jeweils durch die 2,7-Positionen der Phenanthreneinheiten erfolgt, wie über die gestrichelten Bindungen angedeutet. Mögliche Substituenten an den Gruppen R sind für die bessere Übersichtlichkeit im Allgemeinen nicht aufgeführt. Alkyl steht hier allgemein für eine aliphatische Alkylgruppe, Aryl für ein aromatisches oder heteroaromatisches System, wie für R beschrieben. Dabei sind die Beispiele 1 bis 21 Beispiele für Grundgerüst-Einheiten, die Beispiele 22 bis 33 Beispiele für emittierende Einheiten und die Beispiele 34 bis 36 Beispiele für lochleitende Einheiten.

| | | |
|---|---|---|
| 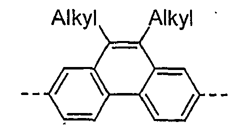 | 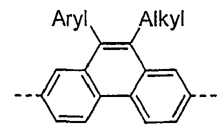 | 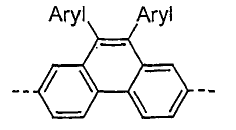 |
| Beispiel 1 | Beispiel 2 | Beispiel 3 |
| 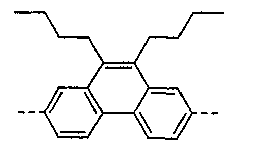 | 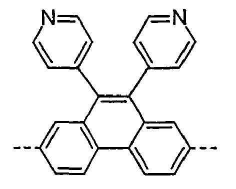 | 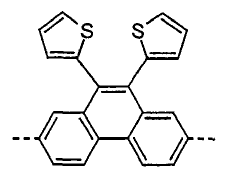 |
| Beispiel 4 | Beispiel 5 | Beispiel 6 |
| 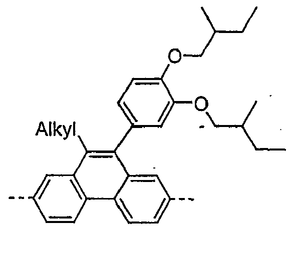 | 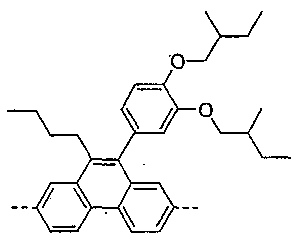 | 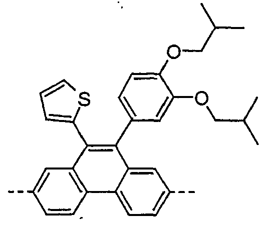 |
| Beispiel 7 | Beispiel 8 | Beispiel 9 |
| 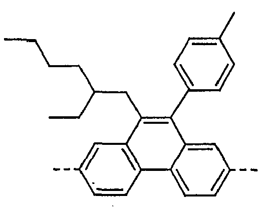 | 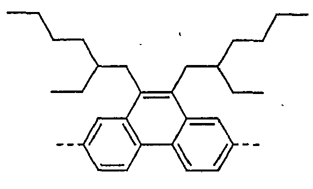 | 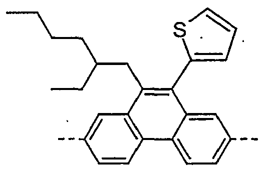 |
| Beispiel 10 | Beispiel 11 | Beispiel 12 |
| 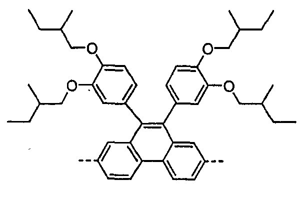 | 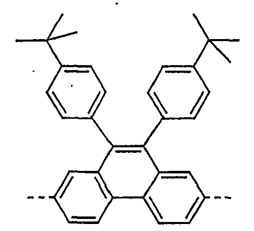 | 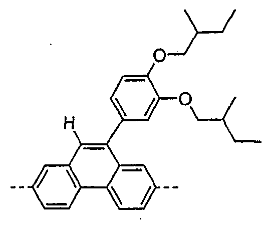 |
| Beispiel 13 | Beispiel 14 | Beispiel 15 |
| 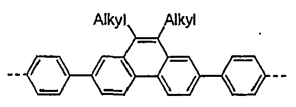 | 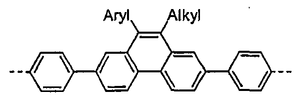 | 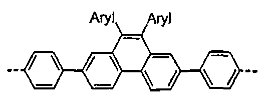 |
| Beispiel 16 | Beispiel 17 | Beispiel 18 |
| 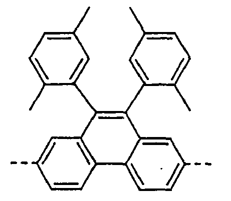 | 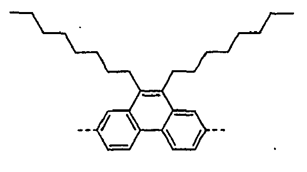 | 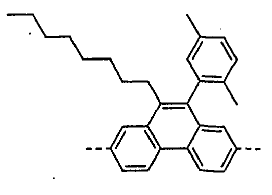 |
| Beispiel 19 | Beispiel 20 | Beispiel 21 |
| 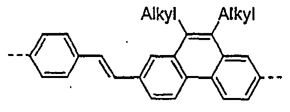 | 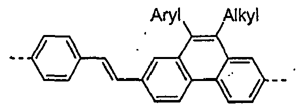 | 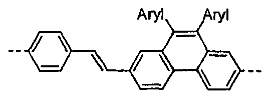 |
| Beispiel 22 | Beispiel 23 | Beispiel 24 |
| 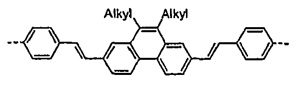 | 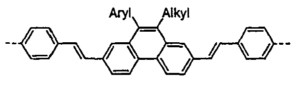 | 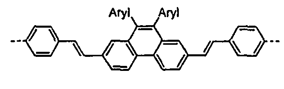 |
| Beispiel 25 | Beispiel 26 | Beispiel 27 |
| 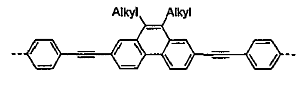 | 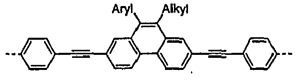 | 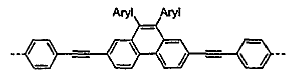 |
| Beispiel 28 | Beispiel 29 | Beispiel 30 |
| 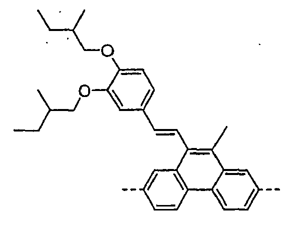 | 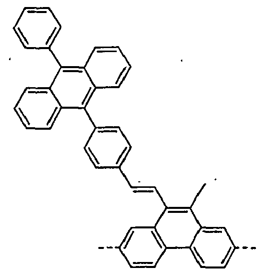 | 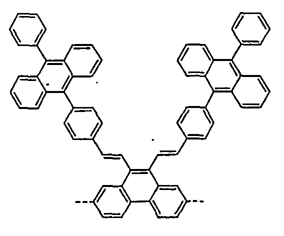 |
| Beispiel 31 | Beispiel 32 | Beispiel 33 |
| | | |
| 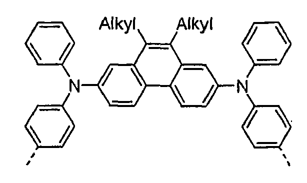 | 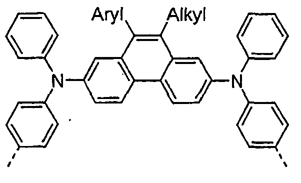 | 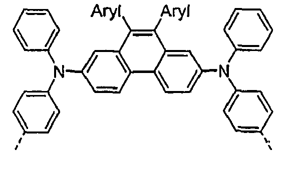 |
| Beispiel 34 | Beispiel 35 | Beispiel 36 |

Die erfindungsgemäßen Polymere sind Homopolymere oder Copolymere.
Erfindungsgemäße Copolymere können dabei neben einer oder mehreren Strukturen gemäß Formel (1) potenziell eine oder mehrere weitere Strukturen, bevorzugt aus den oben genannten Gruppen 1 bis 7, besitzen.
Die erfindungsgemäßen Copolymere können statistische, alternierende oder blockartige Strukturen aufweisen oder auch mehrere dieser Strukturen abwechselnd besitzen. Wie Copolymere mit blockartigen Strukturen erhalten werden können, ist beispielsweise ausführlich in WO 05/014688 beschrieben.
Durch das Verwenden mehrerer verschiedener Strukturelemente können Eigenschaften wie Löslichkeit, Festphasenmorphologie, Farbe, Ladungsinjektions- und -transporteigenschaften, Temperaturstabilität, elektrooptische Charakteristik, etc. eingestellt - werden.

Die erfindungsgemäßen Polymere werden durch Polymerisation von einer oder mehreren Monomersorten hergestellt, von denen mindestens ein Monomer im Polymer zu Einheiten gemäß Formel (1) führt. Entsprechende Polymerisationsreaktionen gibt es prinzipiell viele. Es haben sich hier jedoch einige Typen besonders bewährt, die zu C-C- bzw. zu C-N-Verknüpfungen führen:
(A) Polymerisation gemäß SUZUKI;
(B) Polymerisation gemäß YAMAMOTO;
(C) Polymerisation gemäß STILLE;
(D) Polymerisation gemäß HARTWIG-BUCHWALD.
Wie die Polymerisation nach diesen Methoden durchgeführt werden kann und wie die Polymere vom Reaktionsmedium abgetrennt und aufgereinigt werden können, ist im Detail in WO 04/037887 beschrieben.

Monomere, die in erfindungsgemäßen Polymeren zu Struktureinheiten gemäß Formel (1) führen, sind Phenanthren-Derivate, die in der 9- und/oder 10-Position geeignet substituiert sind und an der 2,7-Position (bzw. in einer geeigneten Position an Y, falls vorhanden) geeignete Funktionalitäten aufweisen, die es erlauben, diese Monomereinheit in das Polymer einzubauen. Diese Monomere sind neu und daher ebenfalls Gegenstand der vorliegenden Erfindung.

Gegenstand der Erfindung sind weiterhin bifunktionelle monomere Verbindungen gemäß Formel (2), 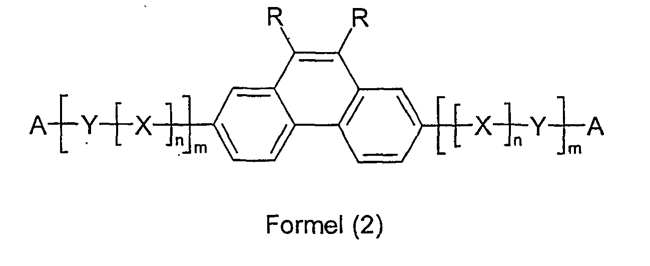 dadurch gekennzeichnet, dass die beiden funktionellen Gruppen A, gleich oder verschieden bei jedem Auftreten sind und ausgewählt sind aus Cl, Br, I, O-Tosylat, 0-Triflat, O-SO₂R², B(OR²)₂ und Sn(R²)₃, wobei R² dieselbe Bedeutung hat, wie in Anspruch 1 beschrieben, und wobei zwei oder mehr Reste R² auch miteinander ein Ringsystem bilden können;
wobei R gleich oder verschieden bei jedem Auftreten, für eine geradkettige, verzweigte oder cyclische Alkylkette mit 2 bis 15 C-Atomen, in der auch ein oder mehrere nicht benachbarte Atome durch N-R¹, O, S, O-CO-O, CO-O, -CH=CH- oder -C=C- ersetzt sein können, mit der Maßgabe, dass die Heteroatome nicht direkt an die Phenanthreri-Einheit gebunden sind, und in der auch ein oder mehrere H-Atome durch F oder CN ersetzt sein können, oder eine aromatische oder heteroaromatische Gruppe mit 4 bis 20 C-Atomen, welche auch durch ein oder mehrere nicht-aromatische Reste R¹ substituiert sein kann, oder eine Kombination aus mehreren dieser Systeme steht;
dabei können die beiden Reste R zusammen auch ein weiteres mono- oder polycyclisches, aromatisches oder aliphatisches Ringsystem bilden, und
wobei die weiteren Symbole und Indizes dieselbe Bedeutung wie in Anspruch 1 beschrieben haben.

Bevorzugt ist A ausgesucht aus Cl, Br, I, O-Tosylat, O-Triflat, O-SO₂R², B(OR²)₂ und Sn(R²)₃, besonders bevorzugt aus Br, I und B(OR²)₂, wobei R² dieselbe Bedeutung hat, wie oben beschrieben, und wobei zwei oder mehr Reste R² auch miteinander ein Ringsystem bilden können.

Die C-C-Verknüpfungen sind bevorzugt ausgewählt aus den Gruppen der SUZUKI-Kupplung, der YAMAMOTO-Kupplung und der STILLE-Kupplung; die C-N-Verknüpfung ist bevorzugt eine Kupplung gemäß HARTWIG-BUCHWALD.

Dabei gilt für bifunktionelle monomere Verbindungen gemäß Formel (2) dieselbe Bevorzugung, wie für Struktureinheiten gemäß Formel (1) oben beschrieben.

Es kann bevorzugt sein, das erfindungsgemäße Polymer nicht als Reinsubstanz, sondern als Mischung (Blend) zusammen mit weiteren beliebigen polymeren, oligomeren, dendritischen oder niedermolekularen Substanzen zu verwenden. Diese können beispielsweise die elektronischen Eigenschaften verbessern, den Transfer vom Singulettzum Triplettzustand beeinflussen oder selber aus dem Singulett- oder aus dem Triplettzustand Licht emittieren. Aber auch elektronisch inerte Substanzen können sinnvoll sein, um beispielsweise die Morphologie des gebildeten Polymerfilms oder die Viskosität von Polymerlösungen zu beeinflussen. Solche Blends sind daher auch Gegenstand der vorliegenden Erfindung.

Gegenstand der Erfindung sind weiterhin Lösungen und Formulierungen aus einem oder mehreren erfindungsgemäßen Polymeren oder Blends in einem oder mehreren Lösungsmitteln. Wie Polymerlösungen hergestellt werden können, ist beispielsweise in WO 02/072714, in WO 03/019694 und in der darin zitierten Literatur beschrieben. Diese Lösungen können verwendet werden, um dünne Polymerschichten herzustellen, zum Beispiel durch Flächenbeschichtungsverfahren (z. B. Spin-coating) oder Druckverfahren (z. B. InkJet Printing).

Die erfindungsgemäßen Polymere können in PLEDs verwendet werden. Diese enthalten Kathode, Anode, Emissionsschicht und gegebenenfalls weitere Schichten, wie z. B. bevorzugt eine Lochinjektionsschicht und gegebenenfalls eine Zwischenschicht zwischen der Lochinjektions- und der Emissionsschicht. Wie PLEDs hergestellt werden können, wird als allgemeines Verfahren ausführlich in WO 04/037887 beschrieben, das entsprechend für den Einzelfall anzupassen ist.
Wie oben beschrieben, eignen sich die erfindungsgemäßen Polymere ganz besonders als Elektrolumineszenzmaterialien in den derart hergestellten PLEDs oder Displays.

Als Elektrolumineszenzmaterialien im Sinne der Erfindung gelten Materialien, die als aktive Schicht in einer PLED Verwendung finden können. Aktive Schicht bedeutet, dass die Schicht befähigt ist, bei Anlegen eines elektrischen Feldes Licht abzustrahlen (lichtemittierende Schicht) und/oder dass sie die Injektion und/oder den Transport der positiven und/oder negativen Ladungen verbessert (Ladungsinjektions- oder Ladungstransportschicht). Es kann sich auch um eine Zwischenschicht zwischen einer Lochinjektionsschicht und einer Emissionsschicht handeln.

Gegenstand der Erfindung ist daher auch die Verwendung eines erfindungsgemäßen Polymers in einer PLED, insbesondere als Elektrolumineszenzmaterial.

Gegenstand der Erfindung ist somit ebenfalls eine PLED mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht und/oder eine Zwischenschicht sein.

Die erfindungsgemäßen Polymere weisen gegenüber den in WO 03/020790 beschriebenen Poly-Spirobifluorenen und Polyfluorenen, beschrieben in WO 02/077060, die hiermit als nächstliegender Stand der Technik genannt werden, folgende überraschenden Vorteile auf:
(1) Es wurde gefunden, dass die erfindungsgemäßen Polymere (bei ansonsten gleicher oder ähnlicher Zusammensetzung) höhere Leuchteffizienzen in der Anwendung aufweisen. Dies gilt besonders für die Copolymere, die blaue Emission zeigen. Dies ist von enormer Bedeutung, da somit entweder gleiche Helligkeit bei geringerem Energieverbrauch erzielt werden kann, was vor allem bei mobilen Applikationen (Displays für Handys, Pager, PDA, etc.), die auf Batterien und Akkus angewiesen sind, sehr wichtig ist. Umgekehrt erhält man bei gleichem Energieverbrauch höhere Helligkeiten, was beispielsweise für Beleuchtungsanwendungen interessant sein kann.
(2) Des Weiteren hat sich überraschend gezeigt, dass wiederum im direkten Vergleich die erfindungsgemäßen Polymere höhere operative Lebensdauern aufweisen, insbesondere im Fall von grün und blau emittierenden PLEDs.
(3) Die Zugänglichkeit und die Erzielbarkeit von Farben ist bei den erfindungsgemäßen Polymeren gleichwertig oder besser im Vergleich zum Stand der Technik. Insbesondere bei blau emittierenden Polymeren wird ein verbesserter Farbort und eine gesättigtere blaue Emission beobachtet.
(4) Die erfindungsgemäßen Polymere sind, auch ohne den Einsatz von elektronenleitenden Comonomeren, gute Elektronenleiter. Elektronenleitende Eigenschaften in Polymeren sind bislang schwierig zu verwirklichen gewesen, da viele Elektronenleiter gemäß dem Stand der Technik für hochwertige Anwendungen nicht ausreichend stabil sind.
(5) Da das neue Polymergrundgerüst gemäß Formel (1) selber zu tiefblauer Emission führt, ist es leicht möglich, emittierende Einheiten einzuführen, die im Polymer immer noch zu blauer Emission führen. Dadurch ist es leicht möglich, Ladungstransport- und Emissionseigenschaften im Polymer zu trennen. Ohne an eine bestimmte Theorie gebunden sein zu wollen, glauben wir, dass dies nötig ist, um stabile Polymere zu erhalten. Dies war jedoch bislang nur schwierig möglich, da das Polymergrundgerüst selber immer gleichzeitig auch emittiert hat.

Im vorliegenden Anmeldetext und auch in den im Weiteren folgenden Beispielen wird auf die Verwendung erfindungsgemäßer Polymere oder Blends in Bezug auf PLEDs und die entsprechenden Displays abgezielt. Trotz dieser Beschränkung der Beschreibung ist es für den Fachmann ohne weiteres erfinderisches Zutun möglich, die erfindungsgemäßen Polymere auch für weitere Verwendungen in anderen elektronischen Devices (Vorrichtungen) zu benutzen, z. B. für organische integrierte Schaltungen (O-ICs), organische Feld-Effekt-Transisforen (O-FETs), organische Dünnfilmtransistoren (O-TFTs), organische Solarzeilen (O-SCs), organische Feld-Quench-Devices (O-FQDs) oder auch organische Laserdioden (O-Laser), um nur einige Anwendungen zu nennen.
Die Verwendung erfindungsgemäßer Polymere in den entsprechenden Vorrichtungen ebenso wie diese Vorrichtungen selbst sind ebenfalls ein Gegenstand der vorliegenden Erfindung.

Weiterhin können die gemachten Ausführungen ebenso auf entsprechende Oligomere oder Dendrimere übertragen werden. Diese sind ebenfalls ein Gegenstand der vorliegenden Erfindung.

Die Erfindung wird durch die nachfolgenden Beispiele näher erläutert, ohne sie darauf einschränken zu wollen.

### Beispiele:

### Beispiel 1: .Synthese von 2,7-Dibrom-9,10-dimethylphenanthren (nicht erfindungsgemäßes Monomer EM1)

### a) Synthese von 2,7-Dibrom-9,10-dihydroxy-9,10-dimethyl-9,10-dihydrophenanthren

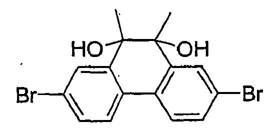

34.3 g (94 mmol) 2,7-Dibromphenanthren-9,10-chinon wurden unter Argon bei -10 °C in 600 mL trockenem THF suspendiert, und es wurden 141 mL (282 mmol) Methylmagnesiumchlorid (2 molare Lösung in THF) so zugetropft, dass die Innentemperatur Q °C nicht überstieg. Anschließend wurde über Nacht bei Raumtemperatur gerührt. Dem Ansatz wurden unter Eiskühlung 50 mL Eisessig zugegeben, und es wurde mit Ethylacetat verdünnt. Nach zweimaligem Waschen mit gesättigter Kochsalzlösung wurde über Natriumsulfat getrocknet und die Lösemittel wurden entfernt. Man erhielt 43.4 g des Produkts, das ohne weitere Aufreinigung für die nächste Stufe eingesetzt wurde.
¹H-NMR (CDCl₃): [ppm] 7.84 (d, ⁴J_{HH} = 2.0 Hz, 2H), 7.52 (d, ³J_{HH} = 8.0 Hz, 2H), 7.46 (dd, ⁴J_{HH} = 2.0 Hz, ³J_{HH} = 8.4 Hz, 2H), 2.10 (s, mit D₂O austauschbar, 2H, OH), 1.30 (s, 6H).

### b) Synthese von 2,7-Dibrom-9-keto-10,10-dimethyl-9,10-dihydrophenanthren

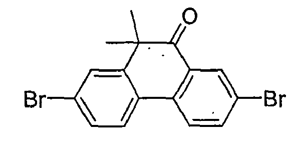

132.8 g (294 mmol) 2,7-Dibrom-9,10-dihydroxy-9,10-dimethyl-9,10-dihydrophenanthren wurden unter Argon in 420 mL Essigsäure und 210 mL Trifluoressigsäure suspendiert und 3 h unter Rückfluss gerührt. Nach Rühren über Nacht bei Raumtemperatur wurde abgesaugt, der Rückstand mit Wasser und Methanol gewaschen, in Toluol gelöst, über Kieselgel filtriert und das Lösemittel entfernt. Man erhielt 89.9 g (80.4 % d. Th.) des Produkts, das ohne weitere Aufreinigung verwendet wurde.
¹H-NMR (CDCl₃): [ppm] 8.17 (d, ⁴J_{HH} = 2.4 Hz, 1 H), 7.77 (m, 3H), 7.63 (d, ⁴J_{HH} = 2.0 Hz, 1H), 7.48 (dd, ³J_{HH} = 8.4 Hz, ⁴J_{HH} = 2.0 Hz, 1 H), 1.53 (s, 6H).

### c) Synthese von 2,7-Dibrom-9-hydroxy-10,10-dimethyl-9,10-dihydrophenanthren

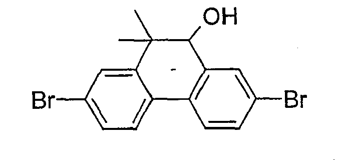

Im ausgeheizten Kolben wurden 2.16 g (57 mmol) Lithiumaluminiumhydrid vorgelegt. Unter Eiskühlung wurden 100 mL THF zugegeben: Danach wurden 43.4 g (114 mmol) 2,7-Dibrom-9-keto-10,10-dimethyl-9,10-dihydrophenanthren in 150 mL THF zugetropft und anschließend unter Rückfluss erhitzt. Über Nacht ließ man auf Raumtemperatur abkühlen, dann wurden vorsichtig 2 mL H₂O zugegeben. Nach 15 min Rühren wurden 2 mL 15 %ige NaOH zugegeben, 15 min gerührt, 6 mL H₂O zugetropft und 15 min gerührt. Der entstandene Feststoff wurde abgesaugt, mit THF gewaschen und das Lösemittel vom Filtrat entfernt. Man erhielt 43.4 g des Produkts, das ohne weitere Aufreinigung eingesetzt wurde.
¹H-NMR (DMSO-d₆): [ppm] 7.77 (m, 2H), 7.64 (d, ⁴J_{HH} = 1.7 Hz, 1 H), 7.56 (d, ⁴J_{HH} = 2.0 Hz, 1H), 7.50 (m, 2H), 5.62 (d, ³J_{HH} = 5.0 Hz, 1 H), 4.35 (d, mit D₂O austauschbar, ³J_{HH} = 5.0 Hz, 1H), 1.23 (s, 3H), 1.03 (s, 3H).

### d) Synthese von 2,7-Dibrom-9,10-dimethylphenanthren (EM1)

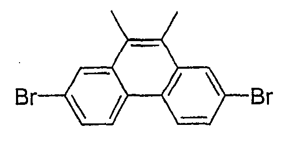

43.4 g (113 mmol) 2,7-Dibrom-9-hydroxy-10,10-dimethyl-9,10-dihydrophenanthren wurden in 610 mL Essigsäure suspendiert. Es wurden 780 mg Iod und 3.5 mL HBr in Essigsäure zugegeben und die Suspension zum Rückfluss erhitzt. Über Nacht ließ man unter Rühren abkühlen. Der Rückstand wurde abgesaugt und mit Wasser und Methanol gewaschen. Man erhielt 35.8 g (87.0 % d. Th.) des Produkts.
¹H-NMR (CDCl₃): [ppm] 8.46 (d, ³J_{HH} = 8.7 Hz, 2H), 8.21 (d, ⁴J_{HH} = 1.7 Hz, 2H), 7.68 (dd, ³J_{HH} = 8.7 Hz, ⁴J_{HH} = 1.7 Hz, 2H), 2.67 (s, 6H).

### Beispiel 2: Synthese von 2,7-Dibrom-9,10-bis(2-ethylhexyl)phenanthren (erfindungsgemäßes Monomer EM2)

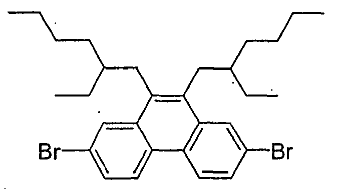

Die Synthese erfolgte in Analogie zu Beispiel 1 unter Verwendung von 2-Ethylhexylmagnesiumchlorid statt Methylmagnesiumchlorid.
¹H-NMR (CDCl₃): [ppm] 8.48 (d, ³J_{HH}= 8.7 Hz, 2H), 8.25 (d, ⁴J_{HH} = 1.3 Hz, 2H), 7.67 (dd, ³J_{HH} = 9.0 Hz, 4J_{HH} = 1.7 Hz, 2H), 3.11 (m, 4H), 1.68 (m, 2H), 1.26 (m, 16H), 0.88 (m, 12H).

### Beispiel 3: Synthese von 2,7-Dibrom-9,10-bis(4-tert-butylphenyl)-phenanthren (erfindungsgemäßes Monomer EM3)

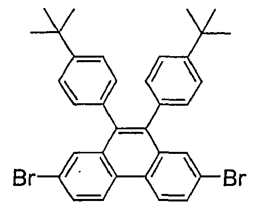

Die Synthese erfolgte in Analogie zu Beispiel 1 unter Verwendung von 4-tert-Butylphenylmagnesiumchlorid statt Methylmagnesiumchlorid. Das Produkt wurde durch mehrfache Umkristallisation aus Toluol und aus Chlorbenzol gereinigt.
¹H-NMR (CDCl₃): [ppm] 8.57 (d, ³J_{HH} = 9.0 Hz, 2H), 7.84 (d, ⁴j_{HH} = 2.0 Hz, 2H), 7.73 (dd, ³J_{HH} = 9.0 Hz, ⁴J_{HH} = 2.0 Hz, 2H), 7.18 (d, ³J_{HH} = 8.4 Hz, 4H), 7.18 (d, ³J_{HH} = 8.7 Hz, 4H), 1.27 (s, 18H).

### Beispiel 4: Synthese von 2,7-Dibrom-9,10-bis(n-octyl)-phenanthren (erfindungsgemäßes Monomer EM4)

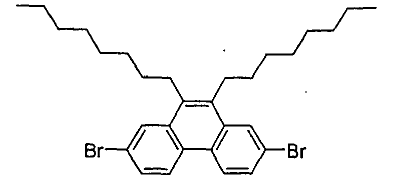

Die Synthese erfolgte in Analogie zu Beispiel 1 unter Verwendung eines GrignardReagenzes aus 1-Octylbromid statt Methylmagnesiumchlorid. Das Produkt wurde durch mehrfache Umkristallisation aus MeOH/Aceton gereinigt.
¹H-NMR (CDCl₃): [ppm] 8.48 (d, ³J_{HH} = 9.0 Hz, 2H), 8.18 (d, ⁴J_{HH} = 2.0 Hz, 2H), 7.67 (dd, ³J_{HH} = 9.0 Hz, ⁴j_{HH} = 2.0 Hz, 2H), 3.01 (m, 4H), 1.66 (m, 4H), 1.54 (m, 4H), 1.43 (m, 4H), 1.32 (m, 12H), 0.91 (t, ³J_{HH} = 7.0 Hz, 6H).

### Beispiel 5: Synthese von 2,7-Bis(boronsäure-ethylenglycolester)-9,10-bis(n-octyl)-phenanthren (erfindungsgemäßes Monomer EM5)

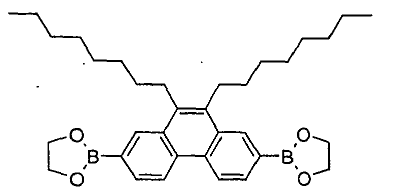

Die Lösung des Grignardreagenzes hergestellt aus 34.4 g (61.4 mmol) **EM4** und 3.14 g (129.1 mmol) in 200 mL trockenem THF wurde bei -75 °C zu einer Lösung aus 20.6 mL (184.2 mmol) Trimethylborat in 70 mL trockenem THF getropft, 3 h bei -75 °C gerührt und dann auf Raumtemperatur gebracht. Die Suspension wurde mit Essigsäureethylester, 10 mL Eisessig und Wasser verdünnt, die organische Phase abgetrennt, zweimal mit Wasser gewaschen, über Natriumsulfat getrocknet und das Lösungsmittel entfernt. Der verbliebene Feststoff wurde in Toluol suspendiert, 6.8 mL (368.4 mmol) wasserfreies Ethylenglycol zugeben und die Suspension am Wasserabscheider 2 h zum starken Sieden erhitzt. Das Lösungsmittel wurde erneut entfernt und der Rückstand aus Ethylacetat bis zu einer Reinheit von 99.9% umkristallisiert.
¹H-NMR (CDCl₃): [ppm] 8.74 (d, ³J_{HH} = 9.0 Hz, 2H), 8.62 (d, ⁴J_{HH} = 2.0 Hz, 2H), 7.97 (dd, ³J_{HH} = 9.0 Hz, ⁴J_{HH} = 2.0 Hz, 2H), 4.46 (s, 8H), 3.20 (m, 4H), 1.72 (m, 4H), 1.59 (m, 4H), 1.44 (m, 4H), 1.32 (m, 12H), 0.91 (t, ³J_{HH} = 7.0 Hz, 6H).

### Beispiel 6: Synthese von N,N'-Bis(4-bromphenyl)-N,N'-bis(4-tert-butylphenyl)-9,10-dimethylphenanthren-2,7-diamin (erfindungsgemäßes Monomer EM6)

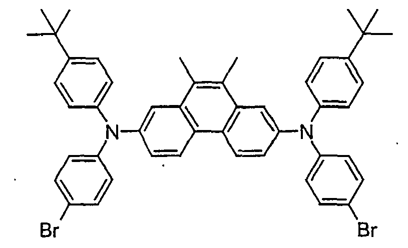

### a) N,N'-Diphenyl-N,N'-bis(4-tert butylphenyl)-9,10-dimethylphenanthren-2,7-diamin

Eine entgaste Lösung aus 30.75 g (84.5 mmol) 2,7-Dibrom-9,10-dimethylphenanthren und 36.0 g (162 mmol) 4-*tert*-Butylphianyl-phenylamin (synthetisiert gemäß *J. Org. Chem.* **2003**, *68*, 452) in 250 ml Toluol wurde 1 h mit N₂ gesättigt. Danach wurde die Lösung zuerst mit 313 mg (1.55 mmol) P(^{t}Bu)₃, dann mit 173 mg (0.76 mmol) Pd(OAc)₂ versetzt; anschließend wurden 9.7 g (101 mmol) NaO^{t}Bu im festen Zustand zugegeben. Die Reaktionsmischung wurde 5 h unter Rückfluss erhitzt. Nach Abkühlen auf Raumtemperatur wurden vorsichtig 1.4 g NaCN und 70 mL Wasser zugesetzt. Die organische Phase wurde mit 4 x 100 mL H₂O gewaschen, über MgSO₄ getrocknet und die Lösemittel im Vakuum entfernt.
Nach chromatographischer Reinigung über Kieselgel erhielt man ein gelbes Öl. Die Ausbeute - bei einer Reinheit von 99.2 % nach HPLC - betrug 59 g (99 % d. Th.). ¹H-NMR (DMSO-d₆, 500 MHz): 1.33 (s, 18H), 2.34 (s, 6H), 6.97-7.89 (m, 10H), 7.95 (d, J = 8.36 Hz, 2H), 7.25 (d, J = 8.7 Hz, 2H), 7.30 (t, J = 7.7 Hz, 4H), 7.36 (d, J = 8.7 Hz,4H), 7.59 (d, J = 2.3 Hz, 2H).

### b) N,N`-Bis(4-bromphenyl)-N,N'-bis(4-tert-butylphenyl)-9,10-dimethylphenanthren-2,7-diamin (EM6)

30 g (54.9 mmol) N,N'-Diphenyl-N,N'-bis(4-*tert*-butylphenyl)-9,10-dimethylphenanthren-2,7-diamin wurden in 600 mL THF vorgelegt. Anschließend tropfte man unter Lichtausschluss bei 0 °C eine Lösung aus 19.03 g (106.0 mmol) NBS, gelöst in 400 mL THF zu, ließ auf Raumtemperatur kommen und rührte weitere 4 h. Anschließend wurde die Mischung mit 600 mL Wasser versetzt und mit CH₂Cl₂ extrahiert. Die organische Phase wurde über MgSO₄ getrocknet und die Lösemittel im Vakuum entfernt. Das Produkt wurde mit Hexan heiß ausgerührt und abgesaugt. Man erhielt 35.1g (94.3 % d. Th.) eines farblosen Feststoffes, welcher nach mehrmaliger Umkristallisation aus Ethylacetat eine HPLC-Reinheit von 99.9 % aufwies.
¹H-NMR (DMSO-d₆, 500 MHz): 1.30 (s, 18H), 2.51 (s, 6H), 6.85 (d, J = 8.7 Hz, 2H), 6.96 (d, J = 8.7 Hz, 3H), 7.09 (d, J = 8.7 Hz, 3H), 7.25 (d, J = 8.76 Hz, 2H), 7.39 (d, J = 8.7 Hz, 4H), 7.45 (d, J = 8.7 Hz, 4H), 7.68 (m, 2H), 8.61 (d, J = 8.8 Hz, 2H).

### Beispiel 7: Synthese von N,N'-Bis(4-brompheny)-N,N'-bis(4-tert-butylphenyl)-9,10-bis(n-octyl)phenanthren-2,7-diamin (erfindungsgemäßes Monomer EM7)

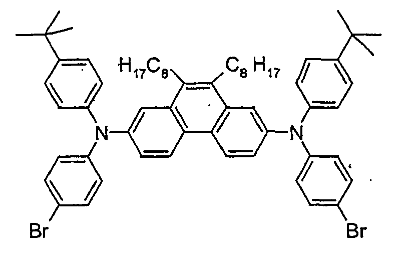

### a) N,N'-Diphenyl-N,N'-bis(4-tert-butylphenyl)-9,10-bis(n-octyl)phenanthren-2,7-diamin

Die Synthese erfolgte in Analogie zu Beispiel 6a), wobei als Edukt 47.3 g (84.5 mmol) 2,7-Dibrom-9,10-bis(*n*-octyl)phenanthren eingesetzt wurden. Nach chromatographischer Reinigung über Kieselgel erhielt man ein gelbes.Öl. Die Ausbeute - bei einer Reinheit von 99.2 % nach HPLC - betrug 50 g (91 % d. Th.).
¹H-NMR (DMSO-d₆, 500 MHz): 0.91 (t, J = 6.7 Hz, 6H),1.34 (s, 18H), 1.36-1.45 (m, 12H), 1.47 (m, 4H), 1.58 (m, 4H), 1.69 (m, 4H), 3.09 (t, J = 8.3 Hz, 4H), 6.96-7.88 (m, 10H), 7.96 (d, J = 8.35 Hz, 2H), 7.23 (d, J = 8.7 Hz, 2H), 7.30 (t, J = 7.7 Hz, 4H), 7.36 (d, J = 8.7 Hz, 4H), 7.59 (d, J = 2.3 Hz, 2H).

### b) N,N'-Bis(4-bromphenyl)-N,N'-bis(4-tert-butylphenyl)-9,10-bis(n-octyl)phenanthren-2,7-diamin (EM7)

Die Synthese erfolgte in Analogie zu Beispiel 6b), wobei als Edukt 55.3 g (54.9 mmol) N,N'-Diphenyl-N,N'-bis(4-*tert*-butylphenyl)-9,10-bis(*n*-octyl)phenanthren-2,7-diamin eingesetzt wurden. Man erhielt 76.1g (90.3 % d. Th.) eines farblosen Feststoffes, welcher nach mehrmaliger Umkristallisation aus Ethylacetat eine HPLC-Reinheit von 99.9 % aufwies.
¹H-NMR (DMSO-d₆, 500 MHz): 0.92 (t, J = 6.7 Hz, 6H),1.33 (s, 18H), 1.36-1.45 (m, 12H), 1.48 (m, 4H), 1.58 (m, 4H), 1.70 (m, 4H), 3.10 (t, J = 8.3 Hz, 4H), 6.85 (d, J = 8.7 Hz, 2H), 6.96 (d, J = 8.7 Hz, 3H), 7.09 (d, J = 8.7 Hz, 3H), 7.25 (d, J = 8.76 Hz, 2H), 7.39 (d, J = 8.7 Hz, 4H), 7.45 (d, J = 8.7 Hz, 4H), 7.68 (m, 2H), 8.61 (d, J = 8.8 Hz, 2H).

### Beispiel 8: 2,7-Bis-[2-(4-bromo-phenyl)-vinyl]-9,10-dioctyl-phenanthren (erfindungsgemäßes Monomer EM8)

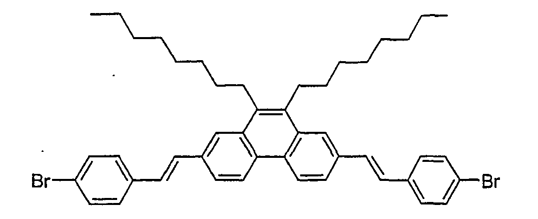

Die Herstellung erfolgte ausgehend von **EM4** durch Herstellung eines Grignardreagenzes analog der Herstellung von **EM5** und anschließender Umsetzung mit 10 Equivalenten DMF. Nach saurer Aufarbeitung, Extraktion und Entfernen des Lösungsmittels wurde der erhaltene Aldehyd nach Charakterisierung durch ¹H-NMR ohne weitere Aufreinigung zum Distilben umgesetzt:
10.7 g (35 mmol) (4-Brombenzyl)-phosphonsäurediethylester wurden in 100 mL trockenem DMF gelöst, unter Schutzgas bei ca. 5°C mit 6.7 g (70 mmol) NaO*^{t}*Bu versetzt, nach 30 min Rührzeit bei 5 °C der Phenanthrenbisaldehyd (7.3 g, 159 mmol) bei max. 5 °C zugegeben und anschließend 1 h bei 5 °C gerührt. Zur Aufarbeitung wurden 20 mL 4 M HCl sowie 50 mL MeOH zugetropft, der entstandene Niederschlag abgesaugt und bis zu einer Reinheit von 99.8 % aus Toluol umkristallisiert.
¹H-NMR (CDCl₃): [ppm] 8.64 (d, ³J_{HH} = 9.0 Hz, 2H), 8.29 (d, ⁴J_{HH} =1.0 Hz, 2H), 7.77 (dd, ³J_{HH} = 9.0 Hz, ⁴J_{HH} = 1.0 Hz, 2H), 7.33 (d, ³J_{HH} = 8.4 Hz, 4H), 7.25 (d, ³J_{HH} = 16 Hz, 2H), 7.11 (d, ³J_{HH} = 16 Hz, 2H), 7.03 (d, ³J_{HH} = 8.4 Hz, 4H), 3.25 (m, 4H), 1.75 (m, 4H), 1.62 (m, 4H), 1.45 (m, 4H), 1.32 (m, 12H), 0.91 (t, ³J_{HH} = 7.0 Hz, 6H).

### Beispiel 9: Polymere P1 bis P3 und Vergleichspolymer V1

Die Polymere wurden gemäß WO 03/048225 synthetisiert. Die weiteren verwendeten Monomere (außer den oben bereits erwähnten) sind im Folgenden abgebildet: 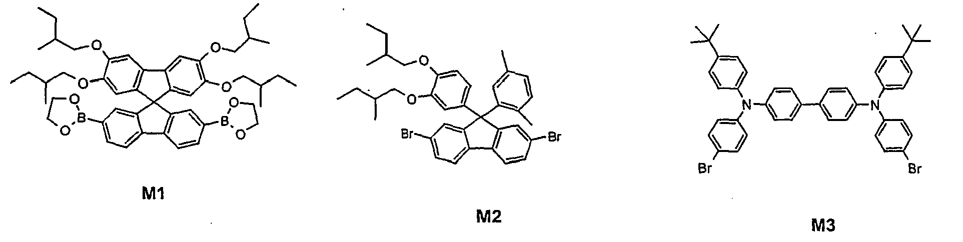 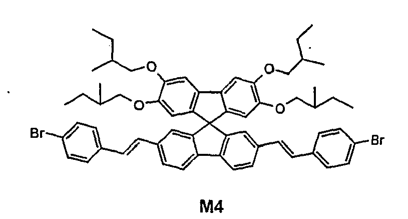 Mit allen Polymeren wurden PLEDs gebaut (nach der allgemeinen Vorschrift gemäß WO 04/037887). Die Zusammensetzung der Polymere und die Ergebnisse der Elektrolumineszenzmessungen sind in Tabelle 1 zusammengefasst:

**Tabelle 1: EL-Daten einiger erfindungsgemäßer Polymere und eines Vergleichspolymers**

| Polymer | Zusammensetzung | Effizienz | Spannung | Farbe |
|---|---|---|---|---|
| **P1** | 50% **M1**, 30% **M2**, 10% **M4**, 10% **EM6** | 4.1 cd/A | 3.7 V | 0.16/0.26 |
| **P2** | 50% **M1**, 30% **M2**, 10% **EM8**,10% **M3** | 4.1 cd/A | 4.0 V | 0.1810.28 |
| **P3** | 50% **M1**, 30% **M2**, 10% **EM8**, 10% **EM6** | 4.1 cd/A | 4.1 V | 0.19/0.31 |
| **V1** | 50% **M1**, 30% **M2**, 10% **M4**, 10% **M3** | 4.1 cd/A | 4.4 V | 0.19/0.33 |

(dabei steht die Effizienz für die maximale Effizienz, die Spannung ist die Spannung, die für eine Helligkeit von 100 cd/m² benötigt wird und die Farbe ist in CIE x/y-Koordinaten angegeben).

Alle Polymere zeigten blaue Lumineszenz mit einer vergleichbaren Effizienz. Dabei war die Spannung der erfindungsgemäßen Polymere niedriger als für das Vergleichspolymer gemäß dem Stand der Technik. Außerdem zeigten die erfindungsgemäßen Polymere eine tiefer blaue Emission als das Vergleichspolymer gemäß dem Stand der Technik und sind somit für die Anwendung besser geeignet. Die Lebensdauer war bei **P1** bis **P3** ca. 5-10 % höher als bei **V1**.

### Beispiel 10: Polymer P4 und Vergleichspolymer V1

Das erfindungsgemäße Polymer **P4** enthält 50 mol% **EM5**, 30 mol% **M2**, 10 mol% **M3** und 10 mol% **M4**. Das Vergleichspolymer **V1** enthält statt des erfindungsgemäßen Monomeres das Monomer **M1**. Mit beiden Polymeren wurden PLEDs gebaut (nach der allgemeinen Vorschrift gemäß WO 04/037887). Das erfindungsgemäße Polymer **P4** zeigte blaue Lumineszenz mit einer Effizienz von 4.3 cd/A, das Vergleichspolymer mit einer Effizienz von 4.1 cd/A. Das erfindungsgemäße Polymer **P4** benötigte eine Spannung von 4.3 V für 100 cd/m², während mit dem Vergleichspolymer **V1** für die gleiche Helligkeit eine Spannung von 4.4 V benötigt wurde. Außerdem zeigte **P4** (CIE x/y 0.18 / 0.30) eine tiefer blaue Emission als V1 (CIE x/y 0.19 / 0.33) und ist somit für die Anwendung besser geeignet als das Vergleichspolymer gemäß dem Stand der Technik. Die Lebensdauer war bei **P4** ca. 30 % höher als bei **V1**.

## Patentansprüche

1. Polymere, enthaltend mindestens 5 mol% Einheiten gemäß Formel (1), 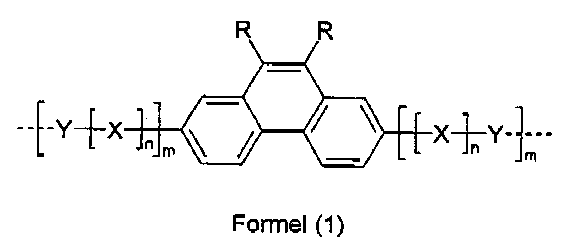 wobei die verwendeten Symbole und Indizes folgende Bedeutung besitzen:
R ist bei jedem Auftreten gleich eine geradkettige, verzweigte oder cyclische Alkylkette mit 1 bis 40 C-Atomen, die durch R¹ substituiert sein kann, und in der auch ein oder mehrere nicht benachbarte C-Atome durch N-R¹, O, S, O-CO-O, CO-O, -CR¹=CR¹- oder -C≡C- ersetzt sein können, mit der Maßgabe, dass die Heteroatome nicht direkt an die Phenanthren-Einheit gebunden sind, und in der auch ein oder mehrere H-Atome durch F, Cl, Br, I oder CN ersetzt sein können, oder ein aromatisches oder heteroaromatisches Ringsystem mit 2 bis 40 C-Atomen, welches auch durch einen oder mehrere Reste R¹ substituiert sein kann, oder eine Kombination aus mehreren dieser Systeme; dabei können die beiden Reste R miteinander auch ein weiteres mono- oder polycyclisches, aliphatisches Ringsystem bilden:
oder verschieden H, eine geradkettige, verzweigte oder cyclische Alkylkette mit 1 bis 40 C-Atomen, die durch R¹ substituiert sein kann, und in der auch ein oder mehrere nicht benachbarte C-Atome durch N-R¹, O, S, O-CO-O, CO-O, -CR¹=CR¹- oder -C=C- ersetzt sein können, mit der Maßgabe, dass die Heteroatome nicht direkt an die Phenanthren-Einheit gebunden sind, und in der auch ein oder mehrere H-Atome durch F, Cl, Br, I oder CN ersetzt sein können, oder ein aromatisches oder heteroaromatisches Ringsystem mit 2 bis 40 C-Atomen, welches auch durch einen oder mehrere Reste R¹ substituiert sein kann, oder eine Kombination aus mehreren dieser Systeme; dabei können die beiden Reste R miteinander auch ein weiteres mono- oder polycyclisches, aliphatisches Ringsystem bilden;
X ist bei jedem Auftreten gleich oder verschieden -CR¹=CR¹-, -C≡C- oder N-Ar;
Y ist bei jedem Auftreten gleich oder verschieden ein bivalentes aromatisches oder heteroaromatisches Ringsystem mit 2 bis 40 C-Atomen, welches durch einen oder mehrere Reste R¹ substituiert oder unsubstituiert sein kann;
R¹ ist bei jedem Auftreten gleich oder verschieden H, eine geradkettige, verzweigte oder cyclische Alkyl- oder Alkoxykette mit 1 bis 22 C-Atomen, in der auch ein oder mehrere nicht benachbarte C-Atome durch N-R², O, S, O-CO-O, CO-O, -CR²=CR²-, -C≡C- ersetzt sein können und in der auch ein oder mehrere H-Atome durch F, Cl, Br, I oder CN ersetzt sein können, oder eine Aryl-, Heteroaryl-, Aryloxy- oder Heteroaryloxygruppe mit 5 bis 40 C-Atomen, welche auch durch einen oder mehrere nicht-aromatische Reste R¹ substituiert sein kann; dabei können auch zwei oder mehrere der Reste R¹ miteinander und/oder mit R ein Ringsystem bilden; oder F, Cl, Br, I, CN, N(R²)₂, Si(R²)₃ oder B(R²)₂;
R² ist bei jedem Auftreten gleich oder verschieden H oder ein aliphatischer oder aromatischer Kohlenwasserstoffrest mit 1 bis 20 C-Atomen;
Ar ist bei jedem Auftreten gleich oder verschieden ein monovalentes aromatisches oder heteroaromatisches Ringsystem mit 2 bis 40 C-Atomen, welches mit R¹ substituiert oder unsubstituiert sein kann;
n ist bei jedem Auftreten gleich oder verschieden 0 oder 1;
m ist bei jedem Auftreten gleich oder verschieden 0, 1 oder 2;
die gestrichelte Bindung bedeutet dabei die Verknüpfung im Polymer; sie soll hier keine Methylgruppe darstellen.

2. Polymere gemäß Anspruch 1, **dadurch gekennzeichnet, dass** es sich um konjugierte oder teilkonjugierte Polymere handelt.

3. Polymere gemäß Anspruch 1 und/oder 2, **dadurch gekennzeichnet, dass** sie neben den Einheiten gemäß Formel (1) noch weitere Strukturelemente enthalten.

4. Polymere gemäß Anspruch 3, **dadurch gekennzeichnet, dass** die weiteren Strukturelementen ausgewählt sind aus den Gruppen der Triarylamin-, Benzidin-, Tetraaryl-para-phenylendiamin-, Triarylphosphin-, Phenothiazin-, Phenoxazin-, Dihydrophenazin-, Thianthren-, Dibenzo-p-dioxin-, Phenoxathiin-, Carbazol-, Azulen-, Thiophen-, Pyrrol- und Furanderivate.

5. Polymere gemäß einem oder mehreren der Ansprüche 1 bis 3, **dadurch gekennzeichnet, dass** die weiteren Strukturelemente ausgewählt sind aus den Gruppen der Pyridin-, Pyrimidin-, Pyridazin-, Pyrazin-, Oxadiazol-, Chinolin-, Chinoxalin-, Benzothiadiazol- und Phenazinderivate, aber auch Triarylborane.

6. Polymere gemäß einem oder mehreren der Ansprüche 1 bis 5, **dadurch gekennzeichnet, dass** die weiteren Strukturelemente Kombinationen von Einzeleinheiten gemäß Anspruch 4 und gemäß Anspruch 5 aufweisen.

7. Polymere gemäß einem oder mehreren der Ansprüche 1 bis 6, **dadurch gekennzeichnet, dass** die weiteren Strukturelemente ausgewählt sind aus der Gruppe der Verbindungen, welche Schweratome mit einer Ordnungszahl von mehr als 36 enthalten.

8. Polymere gemäß einem oder mehreren der Ansprüche 1 bis 7, **dadurch gekennzeichnet, dass** die weiteren Strukturelemente ausgewählt sind aus den Klassen der Carbazol- und überbrücken Carbazoldimereinheiten, Ketone, Phosphinoxide, Sulfoxide, Sulfone und Silan-Derivate.

9. Polymere gemäß einem oder mehreren der Ansprüche 1 bis 8, **dadurch gekennzeichnet, dass** weitere Strukturelemente ausgewählt sind aus den Klassen der 1,4-Phenylen-, 1,4-Naphthylen-, 1,4- oder 9,10-Anthrylen-, 1,6- oder 2,7- oder 4,9-Pyrenylen-, 3,9- oder 3,10- Perylenylen-, 4,4'-Biphenylylen-, 4,4"-Terphenylylen-, 4,4'-Bi-1,1'-naphthylylen-, 4,4'-Tolanylen-, 4,4'-Stilbenylen- oder 4,4"-Bisstyrylarylenderivate.

10. Polymere gemäß einem oder mehreren der Ansprüche 1 bis 9, **dadurch gekennzeichnet, dass** die weiteren Strukturelementen typischerweise als Backbone verwendet werden, ausgewählt aus den Klassen der 4,5-bihydropyrenderivate, 4,5,9,10-Tetrahydro-pyrenderivate, Fluorenderivafie, 9,9'-Spirobifluorenderivate, 9,10-Dihydrophenanthrenderivate, 5,7-Dihydrodibenzo-oxepinderivate und cis- und trans-Indenofluorenderivate.

11. Polymere gemäß einem oder mehreren der Ansprüche 1 bis 10, **dadurch gekennzeichnet, dass** der Anteil der Einheiten gemäß Formel (1) mindestens 10 mol% beträgt.

12. Polymere gemäß einem oder mehreren der Ansprüche 1 bis 11, **dadurch gekennzeichnet, dass** die Polymere außer Einheiten gemäß Formel (1) mindestens zwei Struktureinheiten aus unterschiedlichen Klassen gemäß Anspruch 4 bis 10 enthalten.

13. Polymere gemäß einem oder mehreren der Ansprüche 1 bis 12, **dadurch gekennzeichnet, dass** das Symbol R, gleich oder verschieden bei jedem Auftreten, für eine geradkettige, verzweigte oder cyclische Alkylkette mit 2 bis 15 C-Atomen, in der auch ein oder mehrere nicht benachbarte C-Atome durch N-R¹, O, S, O-CO-O, CO-0, - CH=CH- oder -C≡C- ersetzt sein können, mit der Maßgabe, dass die Heteroatome nicht direkt an die Phenanthren-Einheit gebunden sind, und in der auch ein oder mehrere H-Atome durch F oder CN ersetzt sein können, oder eine aromatische oder heteroaromatische Gruppe mit 4 bis 20 C-Atomen, welche auch durch ein oder mehrere nicht-aromatische Reste R¹ substituiert sein kann, oder eine Kombination aus mehreren dieser Systeme steht; dabei können die beiden Reste R zusammen auch ein weiteres mono- oder polycyclisches, aromatisches oder aliphatisches Ringsystem bilden.

14. Polymere gemäß einem oder mehreren der Ansprüche 1 bis 13, **dadurch gekennzeichnet, dass** das Symbol X, gleich oder verschieden bei jedem Auftreten, für -CH=CH-, -C≡C- oder N-Ar steht.

15. Polymere gemäß einem oder mehreren der Ansprüche 1 bis 14, **dadurch gekennzeichnet, dass** das Symbol Y, gleich oder verschieden bei jedem Auftreten, für ein bivalentes aromatisches oder heteroaromatisches Ringsystem mit 4 bis 25 C-Atomen, welches durch ein oder mehrere Reste R¹ substituiert sein kann, steht.

16. Polymere gemäß einem oder mehreren der Ansprüche 1 bis 15, **dadurch gekennzeichnet, dass** das Symbol Ar, gleich oder verschieden bei jedem Auftreten, für ein monovalentes aromatisches oder heteroaromatisches Ringsystem mit 4 bis 25 Atomen, welches mit R¹ substituiert oder unsubstituiert sein kann, steht.

17. Polymere gemäß einem oder mehreren der Ansprüche 1 bis 16, **dadurch gekennzeichnet, dass** der Index m, gleich oder verschieden bei jedem Auftreten, für 0 oder 1 steht.

18. Polymere gemäß einem oder mehreren der Ansprüche 1 bis 17, **dadurch gekennzeichnet, dass** sie Einheiten gemäß Formel (1) als Grundgerüst enthalten und dass gilt:
n ist bei jedem Auftreten gleich 0.

19. Polymere gemäß einem oder mehreren der Ansprüche 1 bis 17, **dadurch gekennzeichnet, dass** sie Einheiten gemäß Formel (1) als lochtransportierende Einheiten enthalten und dass gilt:
n ist bei jedem Auftreten gleich oder verschieden 0 oder 1, wobei mindestens ein n = 1 ist;
m ist bei jedem Aufteten gleich oder verschieden 0, 1 oder 2, wobei m ungleich 0 ist, wenn das entsprechende n = 1 ist;
X ist bei jedem Auftreten N-Ar.

20. Polymere gemäß einem oder mehreren der Ansprüche 1 bis 17, **dadurch gekennzeichnet, dass** sie Einheiten gemäß Formel (1) als Emitter enthalten und dass gilt:
n ist bei jedem Auftreten gleich oder verschieden 0 oder 1, wobei mindestens ein n = 1 ist;
m ist bei jedem Auftreten gleich oder verschieden 0, 1 oder 2, wobei m ungleich 0 ist, wenn das entsprechende n =1 ist;
X ist bei jedem Auftreten gleich oder verschieden -CR¹=CR¹-, -C≡C- oder N-Ar, wobei mindestens ein X gleich -CR¹=CR¹- oder -C=C- ist.

21. Polymere gemäß einem oder mehreren der Ansprüche 1 bis 20, **dadurch gekennzeichnet, dass** die Einheiten gemäß Formel (1) in den 9,10-Positionen der Phenanthren-Einheiten symmetrisch substituiert sind.

22. Polymere gemäß einem oder mehreren der Ansprüche 1 bis 21, **dadurch gekennzeichnet, dass** sie durch Polymerisation gemäß SUZUKI, Polymerisation gemäß YAMAMOTO, Polymerisation gemäß STILLE oder Polymerisation gemäß HARTWIG-BUCHWALD hergestellt werden.

23. Bifunktionelle monomere Verbindungen gemäß Formel (2), 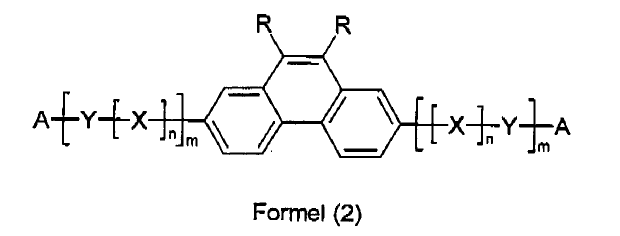 **dadurch gekennzeichnet, dass** die beiden funktionellen Gruppen A, gleich oder verschieden bei jedem Auftreten sind und ausgewählt sind aus Cl, Br, I, O-Tosylat, O-Triflat, O-SO₂R², B(OR²)₂ und Sn(R²)₃, wobei R² dieselbe Bedeutung hat, wie in Anspruch 1 beschrieben, und wobei zwei oder mehr Reste R² auch miteinander ein Ringsystem bilden können:
wobei R gleich oder verschieden bei jedem Auftreten, für eine geradkettige, verzweigte oder cyclische Alkylkette mit 2 bis 15 C-Atomen, in der auch ein oder mehrere nicht benachbarte C-Atome durch N-R¹, O, S, O-CO-O, CO-O, -CH=CH- oder -C≡C- ersetzt sein können, mit der Maßgabe, dass die Heteroatome nicht direkt an die Phenanthren-Einheit gebunden sind, und in der auch ein oder mehrere H-Atome durch F oder CN ersetzt sein können, oder eine aromatische oder heteroaromatische Gruppe mit 4 bis 20 C-Atomen, welche auch durch ein oder mehrere nicht-aromatische Reste R¹ substituiert sein kann, oder eine Kombination aus mehreren dieser Systeme steht;
dabei können die beiden Reste R zusammen auch ein weiteres mono- oder polycyclisches, aromatisches oder aliphatisches Ringsystem bilden, und wobei die weiteren Symbole und Indizes dieselbe Bedeutung wie in Anspruch 1 beschrieben haben.

24. Mischungen (Blends) aus einem oder mehreren Polymeren gemäß einem oder mehreren der Ansprüche 1 bis 22 mit weiteren polymeren, oligomeren, dendritischen oder niedermolekularen Substanzen.

25. Lösungen und Formulierungen aus einem oder mehreren Polymeren oder Blends gemäß einem oder mehreren der Ansprüche 1 bis 22 und/oder 24 in einem oder mehreren Lösungsmitteln.

26. Verwendung eines Polymers oder Blends gemäß einem oder mehreren der Ansprüche 1 bis 22 und/oder 24 oder einer Lösung gemäß Anspruch 25 in polymeren Leuchtdioden.

27. Organisches elektronisches Bauteil mit einer oder mehreren aktiven Schichten, **dadurch gekennzeichnet, dass** mindestens eine dieser aktiven Schichten ein oder mehrere Polymere oder Blends gemäß einem oder mehreren der Ansprüche 1 bis 22 und/oder 24 enthält.

28. Organisches elektronisches Bauteil gemäß Anspruch 27, **dadurch gekennzeichnet, dass** es sich um polymere Leuchtdioden (PLED), organische integrierte Schaltungen (O-IC), organische Feld-Effekt-Transistoren (O-FET), organische Dünnfilmtransistoren (O-TFT), organische Solarzellen (O-SC), organische Feld-Quench-Devices (O-FQDs) oder organische Laserdioden (O-Laser) handelt.

29. Organisches elektronisches Bauteil gemäß Anspruch 28, **dadurch gekennzeichnet, dass** es sich um eine polymere Leuchtdiode handelt.

## Claims

1. Polymers containing at least 5 mol% of units of the formula (1) 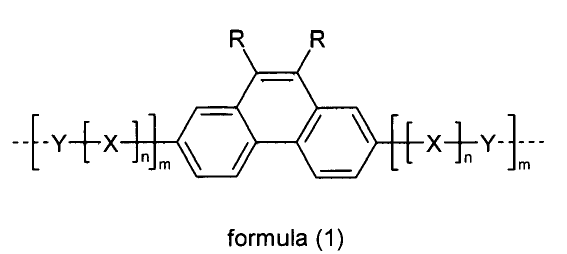 where the symbols and indices used have the following meanings:
R is on each occurrence, identically, a straight-chain, branched or cyclic alkyl chain having 1 to 40 C atoms, which may be substituted by R¹, and in which, in addition, one or more non-adjacent C atoms may be replaced by N-R¹, O, S, O-CO-O, CO-O, -CR¹=CR¹- or -C≡C-, with the proviso that the heteroatoms are not bonded directly to the phenanthrene unit, and in which, in addition, one or more H atoms may be replaced by F, Cl, Br, I or CN, or an aromatic or heteroaromatic ring system having 2 to 40 C atoms, which may also be substituted by one or more radicals R¹, or a combination of a plurality of these systems; the two radicals R here may also form a further mono- or polycyclic, aliphatic ring system with one another;
or, differently, H, a straight-chain, branched or cyclic alkyl chain having 1 to 40 C atoms, which may be substituted by R¹, and in which, in addition, one or more non-adjacent C atoms may be replaced by N-R¹, O, S, O-CO-O, CO-O, -CR¹=CR¹- or -C≡C-, with the proviso that the heteroatoms are not bonded directly to the phenanthrene unit, and in which, in addition, one or more H atoms may be replaced by F, Cl, Br, I or CN, or an aromatic or heteroaromatic ring system having 2 to 40 C atoms, which may also be substituted by one or more radicals R¹, or a combination of a plurality of these systems; the two radicals R here may also form a further mono- or polycyclic, aliphatic ring system with one another;
X is on each occurrence, identically or differently, -CR¹=CR¹-, -C≡C- or N-Ar;
Y is on each occurrence, identically or differently, a divalent aromatic or heteroaromatic ring system having 2 to 40 C atoms, which may be substituted by one or more radicals R¹ or unsubstituted;
R¹ is on each occurrence, identically or differently, H, a straight-chain, branched or cyclic alkyl or alkoxy chain having 1 to 22 C atoms, in which, in addition, one or more non-adjacent C atoms may be replaced by N-R², O, S, O-CO-O, CO-O, -CR²=CR²-, -C≡C- and in which, in addition, one or more H atoms may be replaced by F, Cl, Br, I or CN, or an aryl, heteroaryl, aryloxy or heteroaryloxy group having 5 to 40 C atoms, which may also be substituted by one or more non-aromatic radicals R¹; two or more of the radicals R¹ here may also form a ring system with one another and/or with R; or F, Cl, Br, I, CN, N(R²)₂, Si(R²)₃ or B(R²)₂;
R² is on each occurrence, identically or differently, H or an aliphatic or aromatic hydrocarbon radical having 1 to 20 C atoms;
Ar is on each occurrence, identically or differently, a monovalent aromatic or hetero-aromatic ring system having 2 to 40 C atoms, which may be substituted by R¹ or unsubstituted;
n is on each occurrence, identically or differently, 0 or 1;
m is on each occurrence, identically or differently, 0, 1 or 2;
the dashed bond here denotes the link in the polymer; it is not intended to represent a methyl group here.

2. Polymers according to Claim 1, **characterised in that** they are conjugated or partially conjugated polymers.

3. Polymers according to Claim 1 and/or 2, **characterised in that** they contain further structural elements besides the units of the formula (1).

4. Polymers according to Claim 3, **characterised in that** the further structural elements are selected from the groups of the triarylamine, benzidine, tetraaryl-para-phenylenediamine, triarylphosphine, phenothiazine, phenoxazine, dihydrophenazine, thianthrene, dibenzo-p-dioxin, phenoxathiyne, carbazole, azulene, thiophene, pyrrole and furan derivatives.

5. Polymers according to one or more of Claims 1 to 3, **characterised in that** the further structural elements are selected from the groups of the pyridine, pyrimidine, pyridazine, pyrazine, oxadiazole, quinoline, quinoxaline, benzothiadiazole and phenazine derivatives, but also triarylboranes.

6. Polymers according to one or more of Claims 1 to 5, **characterised in that** the further structural elements have combinations of individual units according to Claim 4 and according to Claim 5.

7. Polymers according to one or more of Claims 1 to 6, **characterised in that** the further structural elements are selected from the group of the compounds which contain heavy atoms having an atomic number of greater than 36.

8. Polymers according to one or more of Claims 1 to 7, **characterised in that** the further structural elements are selected from the classes of the carbazole and bridged carbazole dimer units, ketones, phosphine oxides, sulfoxides, sulfones and silane derivatives.

9. Polymers according to one or more of Claims 1 to 8, **characterised in that** the further structural elements are selected from the classes of the 1,4-phenylene, 1,4-naphthylene, 1,4- or 9,10-anthrylene, 1,6- or 2,7- or 4,9-pyrenylene, 3,9- or 3,10-perylenylene, 4,4'-biphenylylene, 4,4"-terphenylylene, 4,4'-bi-1,1'-naphthylylene, 4,4'-tolanylene, 4,4'-stilbenylene or 4,4"-bisstyrylarylene derivatives.

10. Polymers according to one or more of Claims 1 to 9, **characterised in that** the further structural elements are typically used as backbone, selected from the classes of the 4,5-dihydropyrene derivatives, 4,5,9,10-tetrahydropyrene derivatives, fluorene derivatives, 9,9'-spirobifluorene derivatives, 9,10-dihydrophenanthrene derivatives, 5,7-dihydrodibenzoxepine derivatives and cis- and trans-indenofluorene derivatives.

11. Polymers according to one or more of Claims 1 to 10, **characterised in that** the proportion of the units of the formula (1) is at least 10 mol%.

12. Polymers according to one or more of Claims 1 to 11, **characterised in that**, in addition to units of the formula (1), the polymers contain at least two structural units from different classes according to Claims 4 to 10.

13. Polymers according to one or more of Claims 1 to 12, **characterised in that** the symbol R, identically or differently on each occurrence, stands for a straight-chain, branched or cyclic alkyl chain having 2 to 15 C atoms, in which, in addition, one or more non-adjacent C atoms may be replaced by N-R¹, O, S, O-CO-O, CO-O, -CH=CH- or -C≡C-, with the proviso that the heteroatoms are not bonded directly to the phenanthrene unit, and in which, in addition, one or more H atoms may be replaced by F or CN, or an aromatic or heteroaromatic group having 4 to 20 C atoms, which may also be substituted by one or more non-aromatic radicals R¹, or a combination of a plurality of these systems; the two radicals R here may together also form a further mono- or polycyclic, aromatic or aliphatic ring system.

14. Polymers according to one or more of Claims 1 to 13, **characterised in that** the symbol X, identically or differently on each occurrence, stands for -CH=CH-, -C≡C- or N-Ar.

15. Polymers according to one or more of Claims 1 to 14, **characterised in that** the symbol Y, identically or differently on each occurrence, stands for a divalent aromatic or heteroaromatic ring system having 4 to 25 C atoms, which may be substituted by one or more radicals R¹.

16. Polymers according to one or more of Claims 1 to 15, **characterised in that** the symbol Ar, identically or differently on each occurrence, stands for a monovalent aromatic or heteroaromatic ring system having 4 to 25 C atoms, which may be substituted by R¹ or unsubstituted.

17. Polymers according to one or more of Claims 1 to 16, **characterised in that** the index m, identically or differently on each occurrence, stands for 0 or 1.

18. Polymers according to one or more of Claims 1 to 17, **characterised in that** they contain units of the formula (1) as backbone, and the following applies:
n is on each occurrence equal to 0.

19. Polymers according to one or more of Claims 1 to 17, **characterised in that** they contain units of the formula (1) as hole-transporting units, and the following applies:
n is on each occurrence, identically or differently, 0 or 1, where at least one n = 1;
m is on each occurrence, identically or differently, 0, 1 or 2, where m is not equal to 0 if the corresponding n = 1;
X is on each occurrence N-Ar.

20. Polymers according to one or more of Claims 1 to 17, **characterised in that** they contain units of the formula (1) as emitters, and the following applies:
n is on each occurrence, identically or differently, 0 or 1, where at least one n = 1;
m is on each occurrence, identically or differently, 0, 1 or 2, where m is not equal to 0 if the corresponding n = 1;
X is on each occurrence, identically or differently, -CR¹=CR¹-, -C≡C- or N-Ar, where at least one X is equal to -CR¹=CR¹- or -C≡C-.

21. Polymers according to one or more of Claims 1 to 20, **characterised in that** the units of the formula (1) are symmetrically substituted in the 9,10-positions of the phenanthrene units.

22. Polymers according to one or more of Claims 1 to 21, **characterised in that** they are prepared by SUZUKI polymerisation, YAMAMOTO polymerisation, STILLE polymerisation or HARTWIG-BUCHWALD polymerisation.

23. Bifunctional monomeric compounds of the formula (2) 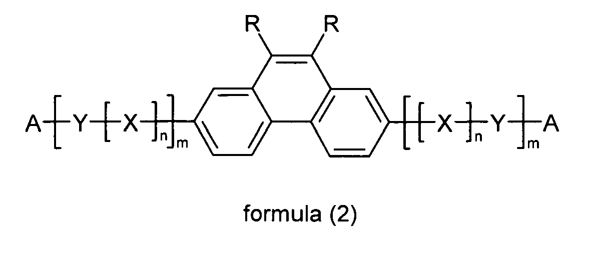 **characterised in that** the two functional groups A, identically or differently on each occurrence, are selected from Cl, Br, I, O-tosylate, O-triflate, O-SO₂R², B(OR²)₂ and Sn(R²)₃, where R² has the same meaning as described in Claim 1, and where two or more radicals R² may also form a ring system with one another;
where R, identically or differently on each occurrence, stands for a straight-chain, branched or cyclic alkyl chain having 2 to 15 C atoms, in which, in addition, one or more non-adjacent C atoms may be replaced by N-R¹, O, S, O-CO-O, CO-O, -CH=CH- or -C=C-, with the proviso that the heteroatoms are not bonded directly to the phenanthrene unit, and in which, in addition, one or more H atoms may be replaced by F or CN, or an aromatic or heteroaromatic group having 4 to 20 C atoms, which may also be substituted by one or more non-aromatic radicals R¹, or a combination of a plurality of these systems; the two radicals R here may together also form a further mono- or polycyclic, aromatic or aliphatic ring system, and where the other symbols and indices have the same meaning as described in Claim 1.

24. Mixtures (blends) of one or more polymers according to one or more of Claims 1 to 22 with further polymeric, oligomeric, dendritic or low-molecular-weight substances.

25. Solutions and formulations of one or more polymers or blends according to one or more of Claims 1 to 22 and/or 24 in one or more solvents.

26. Use of a polymer or blend according to one or more of Claims 1 to 22 and/or 24 or a solution according to Claim 25 in polymeric light-emitting diodes.

27. Organic electronic component having one or more active layers, **characterised in that** at least one of these active layers comprises one or more polymers or blends according to one or more of Claims 1 to 22 and/or 24.

28. Organic electronic component according to Claim 27, **characterised in that** it is polymeric light-emitting diodes (PLEDs), organic integrated circuits (O-ICs), organic field-effect transistors (O-FETs), organic thin-film transistors (O-TFTs), organic solar cells (O-SCs), organic field-quench devices (O-FQDs) or organic laser diodes (O-lasers).

29. Organic electronic component according to Claim 28, **characterised in that** it is a polymeric light-emitting diode.

## Revendications

1. Polymères contenant au moins 5 %mol. d'unités de la formule (1) 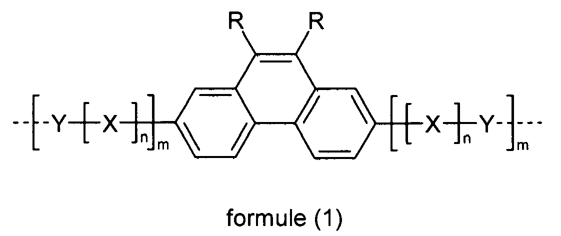 dans laquelle les symboles et indices utilisés ont les significations qui suivent:
R est à chaque occurrence, de façon identique, une chaîne alkyle en chaîne droite, ramifiée ou cyclique ayant 1 à 40 atomes de C, qui peut être substituée par R¹, et dans laquelle, en addition, un ou plusieurs atomes de C non adjacents peuvent être remplacés par N-R¹, O, S, O-CO-O, CO-O, -CR¹=CR¹- ou -C≡C-, étant entendu que les hétéroatomes ne sont pas liés directement à l'unité phénanthrène, et dans lequel, en addition, un ou plusieurs atomes de H peuvent être remplacés par F, Cl, Br, I ou CN, ou un système de cycle aromatique ou hétéro-aromatique ayant 2 à 40 atomes de C, qui peut également être substitué par un ou plusieurs radicaux R¹, ou une combinaison d'une pluralité de ces systèmes, les deux radicaux R peuvent ici également former un système de cycle aliphatique mono- ou polycyclique supplémentaire avec un autre,
ou, de façon différente, H, une chaîne alkyle en chaîne droite, ramifiée ou cyclique ayant 1 à 40 atomes de C, qui peut être substituée par R¹, et dans laquelle, en addition, un ou plusieurs atomes de C non adjacents peuvent être remplacés par N-R¹, O, S, O-CO-O, CO-O, -CR¹=CR¹- ou -C≡C-, étant entendu que les hétéroatomes ne sont pas liés directement à l'unité phénanthrène, et dans lequel, en addition, un ou plusieurs atomes de H peuvent être remplacés par F, Cl, Br, I
ou CN, ou un système de cycle aromatique ou hétéroaromatique ayant 2 à 40 atomes de C, qui peut également être substitué par un ou plusieurs radicaux R¹,
ou une combinaison d'une pluralité de ces systèmes, les deux radicaux R peuvent ici également former un système de cycle aliphatique mono- ou polycyclique supplémentaire avec un autre,
X est à chaque occurrence, de façon identique ou différente, -CR¹=CR¹-C≡C- ou N-Ar;
Y est à chaque occurrence, de façon identique ou différente, un système de cycle aromatique ou hétéroaromatique divalent ayant 2 à 40 atomes de C, qui peut être substitué par un ou plusieurs radicaux R¹ ou non substitué;
R¹ est à chaque occurrence, de façon identique ou différente, H, une chaîne alkyle ou alcoxy en chaîne droite, ramifiée ou cyclique ayant 1 à 22 atomes de C, dans laquelle, en addition, un ou plusieurs atomes de C non adjacents peuvent être remplacés par N-R², O, S, O-CO-O, CO-O, -CR²=CR², -C≡C- et dans laquelle, en addition , un ou plusieurs atomes de H peuvent être remplacés par F, CI, Br, I ou CN, ou un groupe aryle, hétéroaryle, aryloxy ou hétéroaryloxy ayant 5 à 40 atomes de C, qui peut également être substitué par un ou plusieurs radicaux non aromatiques R¹; deux ou plus de deux de ces radicaux R¹ peuvent ici également former un système de cycle avec un autre et/ou avec R; ou F, CI, Br, 1, CN, N(R²)₂, Si(R²)₃ ou B(R²)₂;
R² est à chaque occurrence, de façon identique ou différente, H ou un radical hydrocarbure aliphatique ou aromatique ayant 1 à 20 atomes de C;
Ar est à chaque occurrence, de façon identique ou différente, un système de cycle aromatique ou hétéroaromatique monovalent ayant 2 à 40 atomes de C, qui peut être substitué par R¹ ou non substitué;
n est à chaque occurrence, de façon identique ou différente, 0 ou 1;
m est à chaque occurrence, de façon identique ou différente, 0, 1 ou 2;
la liaison en pointillés représente ici la liaison dans le polymère; elle n'est pas destinée à représenter un groupe méthyle ici.

2. Polymères selon la revendication 1, **caractérisés en ce qu'**ils sont des polymères conjugués ou partiellement conjugués.

3. Polymères selon la revendication 1 et/ou 2, **caractérisés en ce qu'**ils contiennent des éléments structurels supplémentaires en plus des unités de la formule (1).

4. Polymères selon la revendication 3, **caractérisés en ce que** les éléments structurels supplémentaires sont choisis parmi les groupes des dérivés de triarylamine, benzidine, tétraaryl-para-phénylenediamine, triarylphosphine, phénothiazine, phénoxazine, di-hydrophénazine, thianthrène, dibenzo-p-dioxine, phénoxathiyne, carbazole, azulène, thiophène, pyrrole et furanne.

5. Polymères selon une ou plusieurs des revendications 1 à 3, **caractérisés en ce que** les éléments structurels supplémentaires sont choisis parmi les groupes des dérivés de pyridine, pyrimidine, pyridazine, pyrazine, oxadiazole, quinoline, quinoxaline, benzothiadiazole et phénazine, mais également triarylboranes.

6. Polymères selon une ou plusieurs des revendications 1 à 5, **caractérisés en ce que** les éléments structurels supplémentaires ont des combinaisons d'unités individuelles selon la revendication 4 et selon la revendication 5.

7. Polymères selon une ou plusieurs des revendications 1 à 6, **caractérisés en ce que** les éléments structurels supplémentaires sont choisis parmi le groupe des composés qui contiennent des atomes lourds ayant un numéro atomique supérieur à 36.

8. Polymères selon une ou plusieurs des revendications 1 à 7, **caractérisés en ce que** les éléments structurels supplémentaires sont choisis parmi les classes des unités dimère de carbazole et carbazole ponté, des cétones, des oxydes de phosphine, des sulfoxydes, des sulfones et des dérivés de silane.

9. Polymères selon une ou plusieurs des revendications 1 à 8, **caractérisés en ce que** les éléments structurels supplémentaires sont choisis parmi les classes des dérivés de 1,4-phénylène, 1,4-naphtylène, 1,4- ou 9,10-anthrylène, 1,6- ou 2,7- ou 4,9-pyrénylène, 3,9-ou 3,10-pérylénylène, 4,4'-biphénylylène, 4,4"-terphénylylène, 4,4'-bi-1,1'-naphtylylène, 4,4'-tolanylène, 4,4'-stilbénylène ou 4,4"-bisstyrylarylène.

10. Polymères selon une ou plusieurs des revendications 1 à 9, **caractérisés en ce que** les éléments structurels supplémentaires sont utilisés typiquement en tant qu'ossature, choisie parmi les classes des dérivés de 4,5-dihydropyrène, des dérivés de 4,5,9,10-tétrahydropyrène, des dérivés de fluorène, des dérivés de 9,9'-spirobifluorène, des dérivés de 9,10-dihydrophénanthrène, des dérivés de 5,7-dihydrodibenzoxépine et des dérivés de cis- et trans-indénofluorène.

11. Polymères selon une ou plusieurs des revendications 1 à 10, **caractérisés en ce que** la proportion des unités de la formule (1) est d'au moins 10 %mol.

12. Polymères selon une ou plusieurs des revendications 1 à 11, **caractérisés en ce que**, en addition aux unités de la formule (1), les polymères contiennent au moins deux unités structurelles provenant de classes différentes selon les revendications 4 à 10.

13. Polymères selon une ou plusieurs des revendications 1 à 12, **caractérisés en ce que** le symbole R, de façon identique ou différente à chaque occurrence, représente une chaîne alkyle en chaîne droite, ramifiée ou cyclique ayant 2 à 15 atomes de C, dans laquelle, en addition, un ou plusieurs atomes de C non adjacents peuvent être remplacés par N-R¹, O, S, O-CO-O, CO-O, -CH=CH- ou -C≡C-, étant entendu que les hétéroatomes ne sont pas liés directement à l'unité phénanthrène, et dans laquelle, en addition, un ou plusieurs atomes de H peuvent être remplacés par F ou CN, ou un groupe aromatique ou hétéroaromatique ayant 4 à 20 atomes de C, qui peut également être substitué par un ou plusieurs radicaux non aromatiques R¹ ou une combinaison d'une pluralité de ces systèmes, les deux radicaux R peuvent ici également former un système de cycle aromatique ou aliphatique mono- ou polycyclique supplémentaire avec un autre.

14. Polymères selon une ou plusieurs des revendications 1 à 13, **caractérisés en ce que** le symbole X, de façon identique ou différente à chaque occurrence, représente -CH=CH-, -C≡C- ou N-Ar.

15. Polymères selon une ou plusieurs des revendications 1 à 14, **caractérisés en ce que** le symbole Y, de façon identique ou différente à chaque occurrence, représente un système de cycle aromatique ou hétéroaromatique divalent ayant 4 à 25 atomes de C, qui peut être substitué par un ou plusieurs radicaux R¹.

16. Polymères selon une ou plusieurs des revendications 1 à 15, **caractérisés en ce que** le symbole Ar, de façon identique ou différente à chaque occurrence, représente un système de cycle aromatique ou hétéroaromatique monovalent ayant 4 à 25 atomes de C, qui peut être substitué par R¹ ou non substitué.

17. Polymères selon une ou plusieurs des revendications 1 à 16, **caractérisés en ce que** l'index m, de façon identique ou différente à chaque occurrence, représente 0 ou 1.

18. Polymères selon une ou plusieurs des revendications 1 à 17, **caractérisés en ce qu'**ils contiennent des unités de la formule (1) en tant qu'ossature, et ce qui suit s'applique:
n es à chaque occurrence égal à 0.

19. Polymères selon une ou plusieurs des revendications 1 à 17, **caractérisés en ce qu'**ils contiennent des unités de la formule (1) en tant qu'unités de transport de trous, et ce qui suit s'applique:
n est à chaque occurrence, de façon identique ou différente, 0 ou 1, où au moins un n=1;
m est à chaque occurrence, de façon identique ou différente, 0, 1 ou 2, où m n'est pas égal à 0 si le n correspondant = 1;
X est à chaque occurrence N-Ar.

20. Polymères selon une ou plusieurs des revendications 1 à 17, **caractérisés en ce qu'**ils contiennent des unités de la formule (1) en tant qu'émetteurs, et ce qui suit s'applique:
n est à chaque occurrence, de façon identique ou différente, 0 ou 1, où au moins un n=1;
m est à chaque occurrence, de façon identique ou différente, 0, 1 ou 2, où m n'est pas égal à 0 si le n correspondant = 1;
X est à chaque occurrence, de façon identique ou différente, -CR¹=CR¹-, -C≡C- ou N-Ar, où au moins un X est égal à -CR¹=CR¹- ou -C≡C-.

21. Polymères selon une ou plusieurs des revendications 1 à 20, **caractérisés en ce que** les unités de la formule (1) sont substituées de façon symétrique aux positions 9,10 des unités phénanthrène.

22. Polymères selon une ou plusieurs des revendications 1 à 21, **caractérisés en ce qu'**ils sont préparés par polymérisation de SUZUKI, polymérisation de YAMAMOTO, polymérisation de STILLE ou polymérisation de HARTWIG-BUCHWALD.

23. Composés monomériques bifonctionnels de la formule (2) 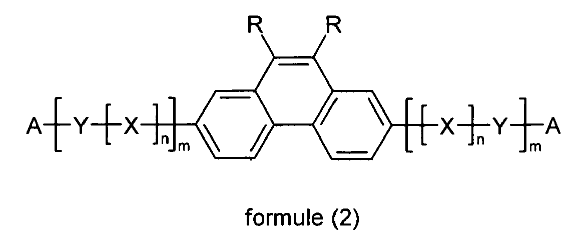 **caractérisés en ce que** les groupes fonctionnels A, de façon identique ou différente à chaque occurrence, sont choisis parmi CI, Br, I, O-tosylate, O-triflate, O-SO₂R², B(OR²)₂ et Sn(R²)₃, où R² a la même signification que celle décrite dans la revendication 1, et où deux ou plus de deux radicaux R² peuvent également former un système de cycle avec un autre;
où R, de façon identique ou différente à chaque occurrence, représente une chaîne alkyle en chaîne droite, ramifiée ou cyclique ayant 2 à 15 atomes de C, dans laquelle, en addition, un ou plusieurs atomes de C non adjacents peuvent être remplacés par N-R¹, O, S, O-CO-O, CO-O, -CH=CH- ou -C≡C-, étant entendu que les hétéroatomes ne sont pas liés directement à l'unité phénanthrène, et dans laquelle, en addition, un ou plusieurs atomes de H peuvent être remplacés par F ou CN, ou un groupe aromatique ou hétéroaromatique ayant 4 à 20 atomes de C, qui peut être substitué par un ou plusieurs radicaux non aromatiques R¹, ou une combinaison d'une pluralité de ces systèmes; les deux radicaux R peuvent ici ensemble également former un système de cycle aromatique ou aliphatique mono- ou polycyclique supplémentaire, et où les autres symboles et indices ont la même signification que celle décrite dans la revendication 1.

24. Mélanges d'un ou plusieurs polymères selon une ou plusieurs des revendications 1 à 22 avec des substances polymériques, oligomériques, dendritiques ou à poids moléculaire faible supplémentaires.

25. Solutions et formulations d'un ou plusieurs polymères ou mélanges selon une ou plusieurs des revendications 1 à 22 et/ou 24 dans un ou plusieurs solvants.

26. Utilisation d'un polymère ou mélange selon une ou plusieurs des revendications 1 à 22 et/ou 24 ou d'une solution selon la revendication 25 dans des diodes émettrices de lumière polymériques.

27. Composant électronique organique ayant une ou plusieurs couches actives, **caractérisé en ce qu'**au moins une de ces couches actives comprend un ou plusieurs polymères ou mélanges selon une ou plusieurs des revendications 1 à 22 et:ou 24.

28. Composant électronique organique selon la revendication 27, **caractérisé en ce qu'**il est des diodes émettrices de lumière polymériques (PLEDs), des circuits intégrés organiques (O-ICs), des transistors à effet de champ organiques (O-FETs), des transistors à film mince organiques (O-TFTs), des cellules solaires organiques (O-SCs), des dispositifs à restriction de champ organiques (O-FQDs) ou des diodes laser organiques (O-lasers).

29. Composant électronique organique selon la revendication 28, **caractérisé en ce qu'**il est une diode émettrice de lumière polymérique.
